# EUROPEAN PATENT APPLICATION

(11) **EP 4 382 128 A1**
(43) Date of publication of application: **12.06.2024**
(21) Application number: 22852073.0
(22) Date of filing: 29.07.2022
(51) Int. Cl.: A61K 47/54, A61K 31/7088, A61K 31/713

(54) **METHOD FOR ENHANCING SUSTAINED RELEASE CAPABILITY OF NUCLEIC ACID DRUG**

(30) Priority: 02.08.2021 CN 202110883137
(71) Applicant: Lnctac Co., Ltd., Chengdu, Sichuan 610200 (CN)
(72) Inventor: SONG, Xu, Chengdu, Sichuan 610200 (CN); ZHAO, Yongyun, Chengdu, Sichuan 610200 (CN); YUAN, Deyu, Chengdu, Sichuan 610200 (CN); ZHANG, Xiaoxia, Chengdu, Sichuan 610200 (CN); ZENG, Miao, Chengdu, Sichuan 610200 (CN)
(74) Representative: KATZAROV S.A.
(86) International application number: PCT/CN2022/109084
(87) International publication number: WO 2023/011360

(57) **Abstract**

The present invention relates to a method for enhancing the sustained release capability of a nucleic acid drug. The method comprises: adding the DNA or RNA of a specific sequence to the 5' end and/or the 3' end of the nucleic acid drug and/or inside same. The enhancing of the sustained release capability of a nucleic acid drug refers to delaying the *in vivo* sustained release time of a nucleic acid drug and increasing the effective action time thereof *in vivo.* The DNA or RNA of the specific sequence is a G-4 chain, and the structure of the G-4 chain contains: Gₓ₁N_{y1}Gₓ₂N_{y2}Gₓ₃N_{y3}Gₓ₄.

## Description

### FIELD OF THE INVENTION

The present invention relates to the fields of biomedicine and biotechnology, and more specifically, relates to a method for enhancing the sustained release ability of a nucleic acid drug.

### BACKGROUND OF THE INVENTION

Sustained release agent refers to a formulation that can release drugs continuously for a long time after administration, so as to achieve a long-term effect. For drugs with short half-life or requiring frequent administration, such formulation can reduce the number of dosage; stabilize blood drug concentration with small fluctuation of blood drug concentration, contributing to reducing toxic and side effects of drug; reduce total dosage and achieve maximum efficacy with minimum dosage. It also has advantages such as small stimulation, long-lasting curative effect, and safety. Thus, more and more attention has been paid to it in clinic.

In order to achieve sustained release of nucleic acid drugs, the methods reported currently are mainly those of using nucleic acid sustained release adjuvant and preparing nucleic acid drugs as polymer nanoparticles. Among them, nucleic acid sustained release adjuvants include Poly(I:C) sustained release adjuvants and sustained release adjuvants prepared with white oil, aluminum stearate and SPAN-80, etc. Polymer nanoparticles include nanoparticles formed by chitosan and PLA-PEG with nucleic acid, etc. However, these preparation methods are complicated, and have not been widely used at present.

G-quadruplex is a high-level structure formed by folding DNA or RNA rich in guanine (G) repeated in series. G-quartet is the structural unit of the quadruplex. Four guanine bases can associate through Hoogsteen hydrogen bonding to form a ring planar. Two or more G-quartets can stack in π-π to form a G-quadruplex. G-quadruplex is a kind of oligonucleotide with stable secondary structure.

At present, there is no relevant report that G-quadruplex or similar structure can play a role in enhancing the sustained release ability of nucleic acids.

Based on the above, the present invention is proposed.

### SUMMARY OF THE INVENTION

The technical problem to be solved by the invention is to provide a method capable of enhancing the sustained release capability of nucleic acid drugs in organisms.

In order to solve the technical problems above, the invention adopts the following technical solutions:
A method for enhancing the sustained release ability of a nucleic acid drug, comprising adding DNA or RNA of a specific sequence at the 5' end and/or 3' end of the nucleic acid drug and/or inside the nucleic acid drug;
enhancing the sustained release ability of a nucleic acid drug means prolonging the sustained release time of the nucleic acid drug *in vivo* to more than 2 days and increasing the effective time of the nucleic acid drug *in vivo* to more than 2 days. Preferably, the sustained release time of the nucleic acid drug *in vivo* is prolonged to 2-20 days, and the effective time of the nucleic acid drug *in vivo* is increased to 2-20 days.

The nucleic acid drug may be an unmodified nucleic acid drug or a chemically modified nucleic acid drug.

Further, the sequence length of the nucleic acid drug is ≥8 nt, preferably 8-5000 nt, more preferably 8-2000 nt, and most preferably 8-1000 nt.

Further, the nucleic acid drug is a single-stranded DNA drug, a double-stranded DNA drug, a single-stranded RNA drug, a double-stranded RNA drug or a nucleic acid analog.

Further, the nucleic acid drug is RNA nucleic acid aptamer, mRNA, ncRNA, antisense oligonucleotide (ASO), DNA nucleic acid aptamer, or other DNA drug.

Further, the ncRNA is miRNA, siRNA, shRNA, saRNA, sgRNA, piRNA, lncRNA, circRNA or another regulatory RNA.

The DNA or RNA of the specific sequence is the DNA or RNA with continuous G bases occurring at intervals. The continuous G bases are repeated 2-10 G bases. The number of intervals of the continuous G bases is 4-16, and the number of repeated G bases in each continuous G bases occurring at intervals is the same or different.

Preferably, the DNA or RNA of the specific sequence also contains a spacer sequence occurring at intervals, which is a sequence of 1-6 arbitrary bases including G or not. The number of intervals of the spacer sequence is 3-15, and each of the spacer sequences occurring at intervals is the same or different.

More preferably, the DNA or RNA of the specific sequence is a G-quadruplex.

The formula of the G-quadruplex comprises Gₓ₁N_{y1}Gₓ₂N_{y2}Gₓ₃N_{y3}Gₓ₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3;
preferably, the G-quadruplex is:
G1 (SEQ ID NO.1): GGGTTGGGTTTGGGTTGGG, or
G1R (SEQ ID NO.9): GGGUUGGGUUUGGGUUGGG.

As a further improvement of the present invention, the DNA or RNA of the specific sequence is a sequence without any modification or a sequence with chemical modification. The chemical modification preferably is MOE modification and thio-modification, and preferably at a 3-end base or 5-end base. More preferably, the sequence of the chemical modification is:

(MOE-G)*(MOE-G)* (MOE-G)* (MOE-T)* TGGGTTTGGGTT(MOE-G)* (MOE-G)* (MOE-G)* ;

wherein, * represents a phosphorothioate linkage; MOE represents that the hydroxyl group (-OH) at the 2' position of the ribose is substituted by methoxyethyl.

The association between DNA or RNA of the specific sequence and a nucleic acid drug is:
DNA of the specific sequence may sustainedly release a DNA drug, DNA of the specific sequence may sustainedly release a RNA drug, RNA of the specific sequence may sustainedly release a DNA drug, or RNA of the specific sequence may sustainedly release a RNA drug.

Preferably, the DNA of the specific sequence is linked with a DNA drug, so as to enhance its sustained release ability; or the RNA of the specific sequence is linked with a RNA drug, so as to enhance its sustained release ability.

The present invention also relates to the use of the G-quadruplex in the preparation of a nucleic acid drug having enhanced sustained release ability.

Enhanced the sustained release ability means prolonging the sustained release time of the nucleic acid drug *in vivo* to more than 2 days and increasing the effective time of the nucleic acid drug *in vivo* to more than 2 days. Preferably, the sustained release time of the nucleic acid drug *in vivo* is prolonged to 2-20 days, and the effective time of the nucleic acid drug *in vivo* is increased to 2-20 days.

The formula of the G-quadruplex comprises Gₓ₁N_{y1}Gₓ₂N_{y2}Gₓ₃N_{y3}Gₓ₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3;
preferably, the G-quadruplex is:
G1 (SEQ ID NO.1): GGGTTGGGTTTGGGTTGGG , or
G1R (SEQ ID NO.9): GGGUUGGGUUUGGGUUGGG.

The present invention also relates to a nucleic acid drug containing G-quadruplex, which knocks down PCSK9 and reduces the Low-Density Lipoprotein (LDL). The nucleic acid drug is an antisense oligonucleotide (ASO) of one or more G-quadruplexes modified at both ends,
(1) the G-quadruplex is a nucleic acid fragment with formula comprising Gₓ₁N_{y1}Gₓ₂N_{y2}Gₓ₃N_{y3}Gₓ₄, wherein:
   G represents guanine;
   x1 - x4 each independently is an integer selected from 2-10, preferably 3-5, and most preferably 3;
   each N independently represents any base (A, C, T, U), preferably T or U;
   y1 - y3 each independently is an integer selected from 1-10, preferably 1-6, and more preferably 1-3;
   preferably, the G-quadruplex is:
   G1 (SEQID NO.1): GGGTTGGGTTTGGGTTGGG, or
   G1R (SEQ ID NO.9): GGGUUGGGUUUGGGUUGGG;
   further, the nucleotide of the G-quadruplex may be chemically modified. Preferably, the chemically modified G-quadruplex is:

      (MOE-G)*(MOE-G)* (MOE-G)* (MOE-T)* TGGGTTTGGGTT(MOE-G)* (MOE-G)* (MOE-G)*
   wherein, * represents a phosphorothioate linkage; MOE represents that the hydroxyl group (-OH) at the 2' position of the ribose is substituted by methoxyethyl;
(2) the ASO is:
   PCSK9-ASO-1 (SEQ ID NO.83): AGCCACGTGGGCAGCAGCCTGTGA
   PCSK9-ASO-2 (SEQ ID NO.84): TTCCACGTGGGCAGCAGCCTGTTT
   PCSK9-ASO-3 (SEQ ID NO.85)_{:} CGTAGACACCCTCACCCCCA
   PCSK9-ASO-4 (SEQ ID NO.86): TTTAGACACCCTCACCCCTT
   PCSK9-ASO-5 (SEQ ID NO.87)_{:} TTTAGACACCCTCACCCCCAATT
   PCSK9-ASO-6 (SEQ ID NO.88): CGTAGACACCCTCACCCCCAAAA
   PCSK9-ASO-7 (SEQ ID NO.89): TTCATCCCGGCCGCTGACCTT
   PCSK9-ASO 8 (SEQ ID NO.90): TTTCCCCAAAGTCCCCTT
   PCSK9-ASO-9 (SEQ ID NO.91): TTCCACGTGGGCAGCAGCCTGTT
   PCSK9-ASO-10 (SEQ ID NO.92): TTGCCACGTGGGCAGCAGCCTGTT
   PCSK9-ASO-11 (SEQ ID NO.93): TTTCAGGGAACCAGGCTT
   PCSK9-ASO-12 (SEQ ID NO.94): TTTCCTCAGGGAACCATT
   PCSK9-ASO-13 (SEQ ID NO.95): TTGCTCCGGCAGCAGATTT
   PCSK9-ASO-14 (SEQ ID NO.96): TTGGGATGCTCTGGGCTT
   PCSK9-ASO-15 (SEQ ID NO.97): TTGCCTGTCTGTGGAATT
   PCSK9-ASO-16 (SEQ ID NO.98)_{:} TTCTGGTCCTCAGGGAACCAGGCCTT.

Preferably, the nucleic acid drug is a nucleic acid shown in any of the following sequences or any combination thereof:
G1/PCSK9-ASO-1 (SEQID NO.59):
GGGTTGGGTTTGGGTTGGGAGCCACGTGGGCAGCAGCCTGTGAGGGTTGGGTTTGGGTTGGG G1/PCSK9-ASO-2 (SEQ ID NO.60):
GGGTTGGGTTTGGGTTGGGTTCCACGTGGGCAGCAGCCTGTTTGGGTTGGGTTTGGGTTGGG GI/PCSK9-ASO-3 (SEQ ID NO.61):
GGGTTGGGTTTGGGTTGGGCGTAGACACCCTCACCCCCAGGGTTGGGTTTGGGTTGGG G1/PCSK9-ASO-4 (SEQ ID NO.62)_{:}
GGGTTGGGTTTGGGTTGGGTTTAGACACCCTCACCCCTTGGGTTGGGTTTGGGTTGGG G1/PCSK9-ASO-5 (SEQ ID NO.63):
GGGTTGGGTTTGGGTTGGGTTTAGACACCCTCACCCCCAATTGGGTTGGGTTTGGGTTGGG G1/PCSK9-ASO-6 (SEQ ID NO.64):
GGGTTGGGTTTGGGTTGGGCGTAGACACCCTCACCCCCAAAAGGGTTGGGTTTGGGTTGGG G1/PCSK9-ASO-7 (SEQ ID NO.65):
GGGTTGGGTTTGGGTTGGGTTCATCCCGGCCGCTGACCTTGGGTTGGGTTTGGGTTGGG Gl/PCSK9-ASO-S (SEQ ID NO.66):
GGGTTGGGTTTGGGTTGGGTTTCCCCAAAGTCCCCTTGGGTTGGGTTTGGGTTGGG G1/PCSK9-ASO-9 (SEQ ID NO.67)_{:}
GGGTTGGGTTTGGGTTGGGTTCCACGTGGGCAGCAGCCTGTTGGGTTGGGTTTGGGTTGGG G1/PCSK9-ASO-10 (SEQ ID NO.68):
GGGTTGGGTTTGGGTTGGGTTGCCACGTGGGCAGCAGCCTGTTGGGTTGGGTTTGGGTTGGG G1/PCSK9-ASO-11 (SEQ ID NO.69):
GGGTTGGGTTTGGGTTGGGTTTCAGGGAACCAGGCTTGGGTTGGGTTTGGGTTGGG G1/PCSK9-ASO-12 (SEQ ID NO.70):
GGGTTGGGTTTGGGTTGGGTTTCCTCAGGGAACCATTGGGTTGGGTTTGGGTTGGG G1/PCSK9-ASO-13 (SEQ ID NO.71)_{:}
GGGTTGGGTTTGGGTTGGGTTGCTCCGGCAGCAGATTTGGGTTGGGTTTGGGTTGGG G1/PCSK9-ASO-14 (SEQ ID NO.72):
GGGTTGGGTTTGGGTTGGGTTGGGATGCTCTGGGCTTGGGTTGGGTTTGGGTTGGG Gl/PCSK9-ASO-15 (SEQ ID NO.73):
GGGTTGGGTTTGGGTTGGGTTGCCTGTCTGTGGAATTGGGTTGGGTTTGGGTTGGG G1/PCSK9-ASO-16 (SEQ ID NO.74):
GGGTTGGGTTTGGGTTGGGTTCTGGTCCTCAGGGAACCAGGCCTTGGGTTGGGTTTGGGTTGGG

By the above technical solutions, the present invention has at least the following advantages:
(1) The inventor found that the DNA or RNA of a specific sequence may enhance the sustained release ability of nucleic acid drugs in organisms. Also, the inventor experimentally verified that the method may enhance the sustained release ability of single-stranded DNA, double-stranded DNA, single-stranded RNA and double-stranded RNA with different sequences and lengths in animals. It proves that the method can enhance the sustained release ability of different forms of nucleic acid drugs in animals and further promote the application of nucleic acid drugs;
(2) By using the method of the present invention, the sustained release and long-term improvement of nucleic acid drugs with a length within 5000 nt may be carried out, and the sustained release effect may be significantly improved. That is, the sustained release time of nucleic acid drugs *in vivo* may be prolonged to about 2-20 days, and the effective time of nucleic acid drugs *in vivo* may be increased to about 2-20 days.

### [Description of terms]

"Nucleic acid", "nucleic acid molecule" and/or "nucleic acid drug".

These terms may be used interchangeably herein, and refer to any DNA, RNA, or DNA/RNA chimera. They may be oligonucleotides or polynucleotides. They may be unmodified or modified RNA or DNA. These terms include but are not limited to, single-stranded and double-stranded DNA, DNA of a mixture of single-stranded and double-stranded regions, single-stranded and double-stranded RNA, RNA of a mixture of single-stranded and double-stranded regions, and hybrid molecules containing DNA and RNA that may be single-stranded or double-stranded or a mixture of single-stranded and double-stranded regions. Nucleotides include adenine (A), guanine (G), thymine (T), cytosine (C), and uracil (U).

The terms "nucleic acid", "nucleic acid molecule" and/or "nucleic acid drug" of the present invention may be used in various applications, such as gene knockdown, gene knockout, gene activation, gene modification, gene editing, gene regulation, protein expression, protein regulation or biological detection or as a nucleic acid drug.

"Nucleic acid modification", "chemical modification" and/or "chemically modified nucleic acid".

These terms may be used interchangeably herein, and refer to any one of the following or combination thereof:
(1) Modification of nucleic acid structure.
   In some embodiments, the nucleic acid (e.g., RNA targeting DNA) of the present invention contains one or more modification(s) (e.g., base modification, skeleton modification, etc.) to provide new or enhanced properties (e.g., improved stability) to the nucleic acid. As known in the art, nucleosides are base-sugar combinations. The base moiety of a nucleoside is generally a heterocyclic base. Purines and pyrimidines are the two most common types of such heterocyclic bases. A nucleotide comprises a nucleoside and a phosphate group covalently linked to the sugar moiety of the nucleoside. For those nucleosides comprising pentofuranose, the phosphate group may be linked to the 2', 3' or 5' hydroxyl moiety of the sugar. In the process of forming nucleic acids, the phosphate groups covalently link nucleosides adjacent to each other to form linear polymerization compounds. Conversely, each end of the linear polymeric compound may be further linked to form a cyclic compound. Linear compounds are generally suitable. In addition, linear compounds may have internal nucleotide-base complementarity and thus may be folded in such a way as to produce fully or partially double-stranded compounds. The phosphate groups within nucleic acids are often referred to as the internucleoside backbone that forms nucleic acids. The normal bond or backbone of RNA and DNA is 3' to 5' phosphodiester linkage.
(2) Modified backbone and modified internucleoside linkage.

Examples of suitable nucleic acids with modifications include nucleic acids with modified backbones or non-natural internucleoside linkages. Nucleic acids (with modified backbones) include those that retain phosphorus atoms in the backbones and those that do not have a phosphorus atom in the backbone.

Suitable modified nucleic acids containing a phosphorus atom therein include, for example, phosphorothioates, chiral phosphorothioates, phosphorodithioates, phosphotriesters, aminoalkylphosphotriesters, methyl and other alkyl phosphates including 3'-alkylene phosphates, 5'-alkylene phosphates, and chiral phosphates, phosphonates, phosphoramidates including 3'-amino phosphoramidate and aminoalkylphosphoramidates, diaminophosphate, thionophosphoramidates, thionoalkylphosphonates, thionoalkylphosphotriesters, selenophosphates and borophosphates having normal 3'-5' linkages, 2'-5'-linked analogs of these, and those having inverted polarity; wherein one or more internucleotide linkages are 3' to 3', 5' to 5' or 2' to 2' linkages. Suitable nucleic acids with reverse polarity contain a single 3' to 3' linkage at the 3' internucleotide linkage, that is, it may be a single inverted nucleoside residue without base (nucleobase is lost or substituted by hydroxyl group). Various salts (such as potassium or sodium salt), mixed salts and free acid forms are also included.

In some embodiments, the nucleic acid of the present invention contains one or more phosphorothioate linkages and/or heteroatom internucleoside linkages, which specifically may be -CH₂-NH-O-CH₂-, -CH₂-N(CH₃)-O-CH₂- (known as methylene (methylimino) or MMI backbone), -CH₂-O-N(CH₃)-CH₂-, -CH₂-N(CH₃)-N(CH₃)-CH₂-, and -O-N(CH₃)-CH₂-CH₂-(wherein the natural phosphodiester internucleoside linkage is represented as -O-P(=O)(OH)-O-CH₂-).

Other backbone modifications also include, for example, nucleic acids having morpholino backbone structures. For example, in some embodiments, the nucleic acid of the invention comprises a six-membered morpholino ring instead of a ribose ring. In some of these embodiments, phosphodiester linkages are replaced by diaminophosphate or other non-phosphodiester internucleoside linkages.

A suitable modified polynucleotide backbones that do not include a phosphorus atom therein have backbones formed by short-chain alkyl or cycloalkyl internucleoside linkages, mixed heteroatoms and alkyl or cycloalkyl internucleoside linkages, or one or more short-chain heteroatomic or heterocyclic internucleoside linkages. These backbones include those having morpholino linkages (partially formed from the sugar moiety of the nucleosides); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulphonate and sulfonamide backbones; amide backbones; and other backbones having mixed N, O, S and CH₂ component parts.

Another backbone modification includes a locked nucleic acid (LNA), in which 2'-hydroxyl is linked to 4' carbon atom of the sugar ring to form a 2'-C, 4'-C-oxymethylene linkage, thereby forming a bicyclic sugar moiety. The chain may be methylene (-CH₂-) (a group bridging 2' oxygen atom and 4' carbon atom), wherein n is 1 or 2 (Singh et al., Chem.Commun., 1998, 4, 455-456). LNA and LNA analogues show very high duplex thermal stability with complementary DNA and RNA (Tm =+3°C to +10°C), stability towards 3'-nucleic acid exogenolysis and good solubility.

The synthesis and preparation of LNA adenine, cytosine, guanine, 5- methyl-cytosine, thymine and uracil monomers, together with their oligomerization and nucleic acid recognition properties, have been described in the prior art (Koshkin et al., Tetrahedron, 1998, 54, 3607-3630).

### (3) Modified sugar moiety.

The nucleic acid of the present invention may also include one or more substituted sugar moieties. Suitable polynucleotides comprise sugar substituents selected from: OH; F; O-, S- or N-alkyl; O-, S- or N-alkenyl; O-, S- or N-alkynyl; or O-alkyl-O-alkyl, wherein the alkyl, alkenyl and alkynyl may be substituted or unsubstituted C1-C10 alkyl or C2-C10 alkenyl and alkynyl. Particularly suitable sugar substituents include O((CH₂)nO)mCH₃, O(CH₂)nOCH₃, O(CH₂)nNH₂, O(CH₂)nCH₃, O(CH₂)nONH₂ and O(CH₂)nON((CH₂)nCH₃)₂, wherein n and m are from 1 to about 10. Other suitable polynucleotides comprise sugar substituents selected from C1-C10 lower alkyl, substituted lower alkyl, alkenyl, alkynyl, alkylaryl, aralkyl, O-alkylaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkylaryl, aminoalkylamino, polyalkylamino, substituted silyl, RNA cleaving groups, reporter groups, intercalators, groups for improving the pharmacokinetic properties of nucleic acids, or groups for improving the pharmacodynamic properties of nucleic acids, and other substituents having similar properties. Suitable modifications include 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504), *i.e.,* an alkoxy-alkoxy group. Another suitable modification includes 2'-dimethylaminooxyethoxy, *i.e.,* a O(CH₂)₂ON(CH₃)₂ group, also known as 2'-DMAOE, as described in the examples hereinafter, and 2'-dimethylaminoethoxyethoxy (also known as 2'-O-dimethyl-amino-ethoxyl-ethyl or 2'-DMAEOE in the art), *i.e.,* 2'-O-CH₂-O-CH₂-N(CH₃)₂.

Other suitable sugar substituents include methoxyl (-O-CH₃), aminopropyloxyl (-OCH₂CH₂CH₂NH₂), allyl (-CH₂-CH=CH₂), -O-allyl (-O-CH₂-CH=CH₂) and fluorine (F). The 2'-sugar substituent may be in the arabinose (upper) position or ribose (lower) position. Suitable 2'-arabinose modification is 2'-F. Similar modifications may also be made at other positions on the oligomeric compound, specifically at the 3' terminal nucleoside or at the 3' position of sugar and the 5' position of the 5' terminal nucleotide in the 2'-5' linked nucleic acid. Oligomeric compound may also have sugar mimetics such as cyclobutyl moieties in place of the pentofuranose.

### (4) Base modification and substitution.

The nucleic acid of the present invention may also include modification or substitution of nucleobase (often referred to in the art simply as "base"). As used herein, "unmodified" or "natural" nucleobases include purine bases adenine (A) and guanine (G), and pyrimidine bases thymine (T), cytosine (C) and uracil (U). Modified nucleobases include other synthetic and natural nucleobases, such as 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halo uracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo (particularly 5-bromo), 5-trifluoromethyl and other 5-substituted uracils and cytosines, 7-methyl guanine and 7-methyl adenine, 2-F-adenine, 2-amino-adenine, 8-azaguanine and 8-azaadenine, 7-deazaguanine and 7-deazaadenine, and 3-deazaguanine and 3-deazaadenine. Other modified nucleobases include tricyclic pyrimidine such as phenoxazine cytidine (1H-pyrimido(5,4-b)(1,4)benzoxazine-2(3H)-one) and phenothiazine cytidine (1H-pyrimido(5,4-b)(1,4)benzothiazine-2(3H)-one), G-clips such as substituted phenazine cytidine (e.g. 9-(2-aminoethoxy)-H-pyrimido(5,4-(b)(1,4)benzoxazine-2(3H)-one), carbazole cytidine (2H-pyrimido(4,5-b)indole-2-one), pyridoindole cytidine (H-pyrido(3',2':4,5)pyrrolo(2,3-d)pyrimidine-2-one).

Heterocyclic base moieties may also include those in which purine or pyrimidine bases are replaced by other heterocycles, such as 7-deazaadenine, 7-deazaguanine, 2-aminopyridine and 2-pyridone. Other nucleobases include those disclosed in The Concise Encyclopedia of Polymer Science and Engineering, pp. 858-859, Kroschwitz, J. L, ed. John Wiley & Sons, 1990, those disclosed by Angewandte Chemie, International edition, 30, 613, 1991, and those disclosed by Sanghvi,YS, Chapter 15, Antisense Research and Applications, pages 289-302, Crooke, S. T. and Lebleu, B., Ed., CRC Press, 1993. Certain of these nucleobases are useful for increasing the binding affinity of the oligomeric compounds. These nucleobases include 5-substituted pyrimidine, 6-azapyrimidine and N-2, N-6 and O-6 substituted purines, including 2-aminopropyladenine, 5-propynyl uracil and 5-propynyl cytosine. 5-methylcytosine substitutions have been shown to increase nucleic acid duplex stability by 0.6°C-1.2°C (Sanghvi et al., Eds., Antisense Research and Applications, CRC Press, Boca Raton, 1993, pages 276-278) and are suitable base substitutions, even more particularly when combined with 2'-O-methoxyethyl sugar modifications, for example.

### (5) Conjugate modification.

Another possible modification of the nucleic acid of the present invention involves chemically linking one or more moieties or conjugates that enhance the activity, cellular distribution or cellular absorption of the nucleic acid to a polynucleotide. These moieties or conjugates may include conjugate groups that covalently bind functional groups such as primary hydroxyl groups or secondary hydroxyl groups. Conjugate groups include, but are not limited to, intercalators, reporter molecules, polyamines, polyamides, polyethylene glycols, polyethers, groups for enhancing the pharmacodynamic properties of oligomers, and groups for enhancing the pharmacokinetic properties of oligomers. Suitable conjugate groups include, but are not limited to, cholesterol, lipids, phospholipids, biotin, phenazine, folate, phenanthridine, anthraquinone, acridine, fluorescein, rhodamine, coumarin and dyes. The groups for enhancing pharmacodynamic properties include groups for improving absorption, enhancing resistance to degradation, and/or enhancing sequence-specific hybridization with target nucleic acids. The groups for enhancing pharmacokinetic properties include groups for improving the absorption, distribution, metabolism or excretion of the nucleic acid of the present invention. Conjugate moieties include, but are not limited to:

lipid moieties such as a cholesterol moiety (Letsinger et al., Proc. Natl. Acid. Sci. USA, 1989, 86, 6553-6556), cholic acid (Manoharan et al., Bioorg. Med. Chem. Let., 1994, 4, 1053-1060), a thioether, *e.g.,* beryl-S-tritylthiol (Manoharan et al., Ann. N.Y. Acad. Sci., 1992, 660, 306-309; Manoharan et al., Bioorg. Med. Chem. Let., 1993, 3, 2765-2770), thiocholesterol (Oberhauser et al., Nucl. Acids Res., 1992, 20, 533-538), aliphatic chain, *e.g.,* dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75, 49-54), phospholipid, *e.g.,* di-hexadecyl-rac-glycerol or triethyl-ammonium 1,2-di-O-hexadecyl-rac-glycero-3-phosphonate (Manoharan et al., Tetrahedron Lett., 1995, 36, 3651-3654; Shea et al., Nucl. Acids Res., 1990, 18, 3777-3783), polyamine or polyethylene glycol chain (Manoharan et al., Nucleosides & Nucleotides, 1995, 14, 969-973), or adamantane acetic acid (Manoharan et al., Tetrahedron Lett., 1995, 36, 3651-3654), palmityl moiety (Mishra et al., Biochim. Biophys. Acta, 1995, 1264, 229-237), or octadecylamine or hexylamino-carbonyloxycholesterol moiety (Crooke et al., J. Pharmacol. Exp. Ther., 1996, 277, 923-937).

"G-quadruplex".

G-quadruplex is a high-level structure formed by folding DNA or RNA rich in guanine (G) repeated in series. G-quartet is the structural unit of the quadruplex. Four guanine bases can associate through Hoogsteen hydrogen bonding to form a ring planar. Two or more G-quartets can stack in π-π to form a G-quadruplex. G-quadruplex is a kind of nucleic acid with stable secondary structure (see Zahler, A.M., Williamson, J.R., Cech, T.R. & Prescott. D.M. Suppression of Telomerase by G-quarter DMA Structures. Nature 350, 718-720 (1991), which is incorporated herein by reference for all purposes). G-quadruplex is further stabilized by the presence of a cation, especially potassium, which is located in the central channel between each pair of quartets. G-quadruplex may be formed by DNA, RNA, LNA and PNA, and may be intramolecular, bimolecular or tetramolecular G-quadruplex. The structure may be described as parallel or antiparallel, depending on the direction of the strands forming the quartet or part thereof (see, for example, Parkinson G N, Lee M P H, Neidle S, Crystal structure of parallel quadruplexes from human telomeric DNA. Nature 417 (6891): 876-880 (2002); Wang Y, Patel D J, Solution structure of the human telomeric repeat d[AG3(T2AG3)3] G-tetraplex. Structure 1(4): 263-282 (1993); and Dai J. Carver M., Yang D,. Polymorphism of human telonieric quadruples structures. Biochimie. 90(8): 1172-1183 (2008), all of which are incorporated herein by reference for all purposes).

In some embodiments, the nucleic acid molecule having G-quadruplex formula of the present invention comprises: Gx₁Ny₁Gx₂Ny₂Gx₃Ny₃Gx₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3.

In some embodiments, the G-quadruplex of the present invention may be an intramolecular, bimolecular or tetramolecular G-quadruplex. In some embodiments, the G-quadruplex of the present invention may comprise cations, such as monovalent cations such as potassium, sodium ions, etc., and divalent cations such as magnesium, calcium, zinc, copper ions, etc.

"Conjugate", "add" and "connect".

In the present invention, the terms "conjugate", "add" and "connect" are used interchangeably, and all refer to connecting or inserting specific sequence fragments through chemical bonds.

"Pharmaceutically acceptable carrier".

Any and all solvents, dispersion media, coating agents, antibacterial and antifungal agents, isotonic agents, absorption retarders and the like that are compatible with drug administration are included. The use of such carriers and reagents is well known in the art. Unless being incompatible with the substances provided herein, any conventional carriers or reagents is considered to be used in the composition.

### BRIEF DESCRIPTION OF THE DRAWINGS

Fig. 1 shows results of evaluating the sustained release of aptamer enhanced by the method of the present invention in animals;
Fig. 2 shows results of evaluating the sustained release of aptamer enhanced by the method of the present invention in the organs of mice;
Fig. 3 shows results of evaluating the sustained release of double-stranded DNA (dsDNA) enhanced by the method of the present invention in animals;
Fig. 4 shows results of evaluating the sustained release of single-stranded RNA (ssRNA) enhanced by the method of the present invention in animals;
Fig. 5 shows results of evaluating the sustained release of double-stranded RNA (dsRNA) enhanced by the method of the present invention in animals;
Fig. 6 shows results of evaluating the sustained release of antisense oligonucleotide (ASO) enhanced by the method of the present invention in animals;
Fig. 7 shows results of evaluating different G-quadruplexes in enhancing the ability of sustained release of nucleic acids in animals;
Fig. 8 shows results of evaluating sequences modified based on G1 in enhancing the ability of sustained release of nucleic acids in animals;
Fig. 9 shows evaluation of the sustained release effects of G1 at 5' end/and/or 3' end and G1 contained inside and outside (9A), and different types of G1 on different types of nucleic acids (9B) (*in vivo* results of animals);
Fig. 10 shows results of evaluating the ability of antisense oligonucleotide targeting PCSK9 sustainedly released by using the method of the present invention in knocking down PCSK9 (A) and reducing LDL (B) for a long time in animals, and the effect of each G1/PCSK9-ASO in knocking down PCSK9 mRNA in hepatocellular carcinoma cell HCC97H (C).
Fig. 11 shows that the antitumor effect of G1-Aptamer2 (aptamer against FGL-1) was better than that of unmodified Aptamer2.
Fig. 12 shows that the mRNA content of fluorescent protein in animals injected with G1-dsDNA2 (dsDNA 2 of GFP) was significantly higher than that in the control group.
Fig. 13 shows that the fluorescence maintenance time could reach 48 hours in animals injected with G1-mRNA1 (firefly luciferase as a marker gene mRNA).
Fig. 14 shows that the regulation time of HGB1 increased significantly after the animals were injected with G1-ncRNA1 (ncRNA1 for BGLT3).

### DETAILED DESCRIPTION OF THE EMBODIMENTS

Unless otherwise specified, the materials used in the examples herein are all commercially available, and various specific experimental methods used for experiments are conventional experimental methods in the art or are performed according to the steps and conditions suggested by the manufacturer, and may be routinely determined by those skilled in the art as required.

### Example 1. Results of evaluating the sustained release of aptamer enhanced by the method of the present invention in animals.

In order to evaluate the ability of sustained release of aptamer enhanced by the method of the present invention in animals, the nucleic acid aptamer enhanced by the method was labeled with cy5.5. G1 sequences were added at both ends of CD35 sequence respectively, and Cy5.5 was used to form (Cy5.5-G1-CD35).

Firstly, the ability of specific DNA (G1) without any modification to enhance the sustained release of aptamer was tested. Before injection, Cy5.5-G1-CD35 was diluted with normal saline. The mice were weighed and an injection dose of 2 mg/kg was administered to the mice with abdominal depilation. The mice were injected subcutaneously by the following steps: grabbing and fixing the mice, wiping the skin between the two hind limbs with alcohol cotton balls, extracting the medicine according to the weight with the syringe, then gently piercing the skin with the needle inclined upward, and injecting slowly after running the needle subcutaneously about 1-2 cm along the skin. After successful subcutaneous injection, the skin of mice at the injection site would bulge, and the needle would be pulled out smoothly in the opposite direction of needle entry to complete the injection. *In vivo* imaging was performed on Day 0, Day 1, Day 3, Day 5, Day 10 and Day 20 post-injection, respectively, to observe the distribution of Cy5.5-G1-CD35 after subcutaneous injection.

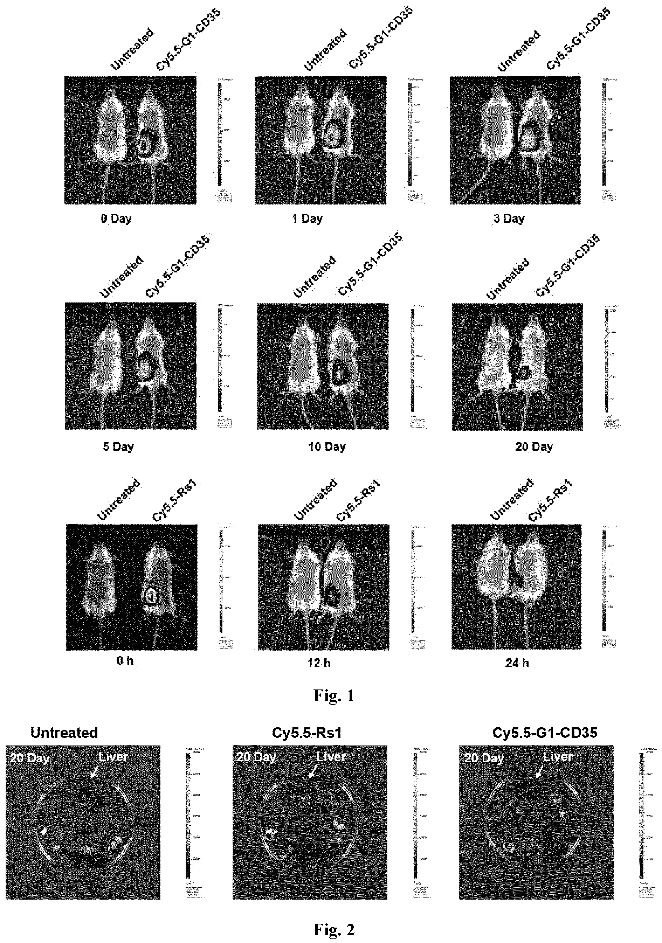

The results (**Fig. 1**) showed that the fluorescence could still be detected on Day 20 after the injection of Cy5.5-labeled Cy5.5-G1-CD35 adopting the method, and the fluorescence on Day 20 was stronger than that of Cy5.5-labeled Cy5.5-Rs1 which did not adopt the enhancement method (in the RS1 sequence, using a random sequence of the same length as the G1 sequence in place of the G1 sequence and adding it at both ends of CD35, as a control) at 24 hours.

Subsequently, the mice were dissected and the fluorescence intensity of the organs was detected. The results (**Fig. 2**) showed that fluorescence could be detected in the liver of mice injected with Cy5.5-G1-CD35, but there was no fluorescence in the control mice, indicating that the aptamer enhanced by the method had good sustained release ability in animals.

In addition, the ability of sustained release of aptamer enhanced by chemically modified specific DNA (G1^{M}) in animals was tested. By using the same injection method, the sustained release Cy5.5-labeled Cy5.5-*G1-CD35 adopting the method for sustained release was injected, and the fluorescence could still be detected on Day 20. Moreover, the fluorescence on Day 20 was stronger than that of Cy5.5-labeled Cy5.5-Rs1 which did not adopt the enhancement method at 24 hours. It indicated that the sustained release ability of the aptamer in animals could also be enhanced by using chemically modified specific DNA.

Wherein:
G1 (SEQID NO.1)_{:}
   GGGTTGGGTTTGGGTTGGG
the chemically modified G1 used in this example is G1^{M}, and its sequence formula and modification are as follows:

   (MOE-G)*(MOE-G)* (MOE-G)* (MOE-T)* TGGGTTTGGGTT(MOE-G)* (MOE-G)* (MOE-G)*
* represents a phosphorothioate linkage; MOE represents that the hydroxyl group (-OH) at the 2' position of ribose is replaced by methoxyethyl;
   CD35 (SEQ ID NO.2) : 
GCTGTGTGACTCCTGCAAAGTGTGGACAACTTCCCACGGAGGAATTCCCGTATCTAAAGGTGCA
GCTGTATCTTGTCTCC ;
Rs1 (SEQ ID NO.3) (using a random sequence of the same length as the G1 sequence in place of the G1 sequence and adding it at both ends of CD35):

GCTGTATCTTGTCTCCTCA
GCTGTGTGACTCCTGCAAAGTGTGGACAACTTCCCACGGAGGAATTCCCGTATCTAAAGGTGCA
GCTGTATCTTGTCTCC
GCTGTATCTTGTCTCCTCA。

### Example 2. Results of evaluating the sustained release of double-stranded DNA (dsDNA) enhanced by the method of the present invention in animals.

In order to evaluate the ability of sustained release of dsDNA enhanced by the method of the present invention in animals, the dsDNA enhanced by the method was labeled with cy5.5. G1 sequences were added at both ends of CD35 sequence respectively, and Cy5.5 was used to form Cy5.5-G1-dsDNA1.

Cy5.5-G1-dsDNA1 was diluted with normal saline before injection. The mice were weighed and an injection dose of 2 mg/kg was administered to the mice with abdominal depilation. The mice were injected subcutaneously by the following steps: grabbing and fixing the mice, wiping the skin between the two hind limbs with alcohol cotton balls, extracting the medicine according to the weight with the syringe, then gently piercing the skin with the needle inclined upward, and injecting slowly after running the needle subcutaneously about 1-2 cm along the skin. After the successful subcutaneous injection, the skin of mice at the injection site would bulge, and the needle would be pulled out smoothly in the opposite direction of needle entry to complete the injection. *In vivo* imaging was performed at different time points after injection to observe the distribution of Cy5.5-G1-dsDNA1 after subcutaneous injection.

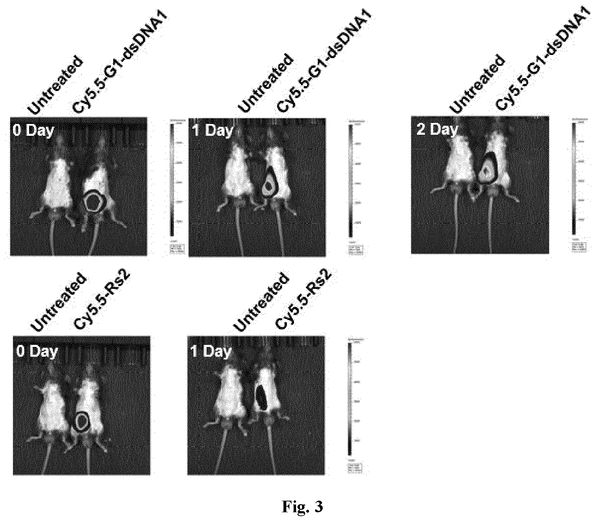

The results (**Fig. 3**) showed that obvious fluorescence could still be detected on Day 2 after the injection of Cy5.5-labeled Cy5.5-G1-dsDNA1 adopting the present method for sustained release, while the fluorescence could hardly be detected in the case of Cy5.5-labeled Cy5.5-Rs1 which did not adopt the enhancement method. It indicated that the dsDNA enhanced by the method still had good sustained release ability in animals.

dsDNA1:
(SEQ ID NO.4): 
5'-GCTGTGTGACTCCTGCAAAGTGTGGACAACTTCCCACGGAGGAATTCCCGTATCTAAAGGTGC
AGCTGTATCTTGTCTCC
(SEQ ID NO.5) 
5'-GGAGACAAGATACAGCTGCACCTTTAGATACGGGAATTCCTCCGTGGGAAGTTGTCCACACTT
TGCAGGAGTCACACAGC
Rs2:
   (SEQ ID NO.6)_{:} 
5'- GCTGTATCTTGTCTCCTCA
GCTGTGTGACTCCTGCAAAGTGTGGACAACTTCCCACGGAGGAATTCCCGTATCTAAAGGTGCA
GCTGTATCTTGTCTCC
GCTGTATCTTGTCTCCTCA
   (SEQ ID NO.7):

5'- TGAGGAGACAAGATACAGC
GGAGACAAGATACAGCTGCACCTTTAGATACGGGAATTCCTCCGTGGGAAGTTGTCCACACTTTG
CAGGAGTCACACAGC
TGAGGAGACAAGATACAGC

### Example 3. Results of evaluating the sustained release of ssRNA enhanced by the method of the present invention in animals.

In order to evaluate the ability of sustained release of ssRNA enhanced by the method of the present invention in animals, the ssRNA enhanced by the method was labeled with cy5.5. G1R sequences were added at both ends of CD35 sequence respectively, and Cy5.5 was used to form Cy5.5-G1-ssRNA1.

Cy5.5-G1R-ssRNA1 was diluted with normal saline before injection. The mice were weighed. The mice were weighed and an injection dose of 2 mg/kg was administered to the mice with abdominal depilation. The mice were injected subcutaneously by the following steps: grabbing and fixing the mice, wiping the skin between the two hind limbs with alcohol cotton balls, extracting the medicine according to the weight with the syringe, then gently piercing the skin with the needle inclined upward, and injecting slowly after running the needle subcutaneously about 1-2 cm along the skin. After the successful subcutaneous injection, the skin of mice at the injection site would bulge, and the needle would be pulled out smoothly in the opposite direction of needle entry to complete the injection. *In vivo* imaging was performed at different time points after injection to observe the distribution of Cy5.5-G1R-ssRNA1 after subcutaneous injection and its degradation in mice.

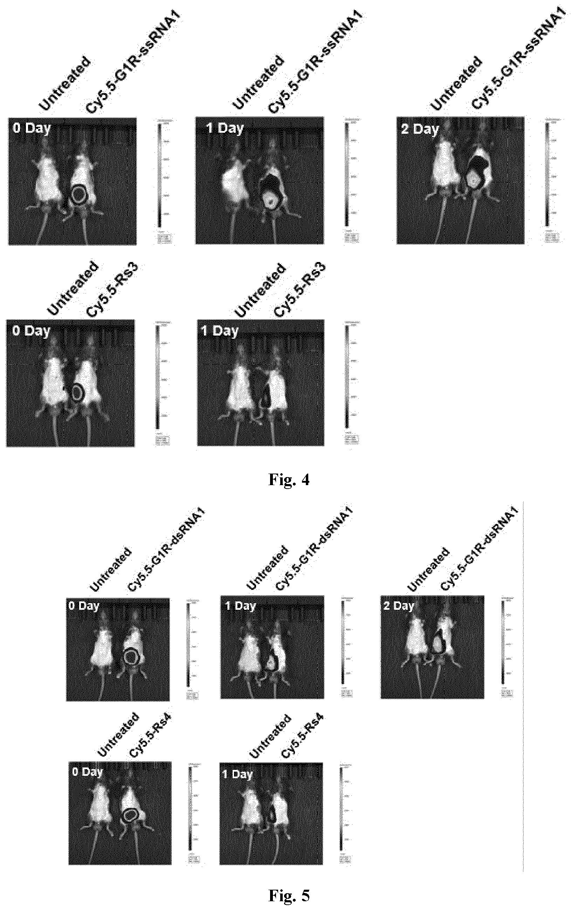

The results (**Fig. 4**) showed that obvious fluorescence could still be detected on Day 2 after the injection of Cy5.5-labeled Cy5.5-G1R-ssRNA1 adopting the present method for sustained release, while the fluorescence could hardly be detected in the case of Cy5.5-labeled Cy5.5-Rs3 without sustained release. It indicated that the ssRNA enhanced by the method still had good sustained release ability in animals.

The sequence information is as follows:
ssRNA1 (SEQIDNO.8): UUGAAUGUAGAGAUGCGGUGG
G1R (SEQID NO.9): GGGUUGGGUUUGGGUUGGG
Rs3 (SEQ ID NO.10)_{:} GCUGUAUCUUGUCUCCUCA

UUGAAUGUAGAGAUGCGGUGG
GCUGUAUCUUGUCUCCUCA

### Example 4. Results of evaluating the sustained release of dsRNA enhanced by the method of the present invention in animals.

In order to evaluate the ability of sustained release of dsRNA enhanced by the method of the present invention in animals, the dsRNA enhanced by the method was labeled with cy5.5. G1R sequences were added at both ends of CD35 sequence respectively, and Cy5.5 was used to form Cy5.5-G1R-dsRNA1.

Cy5.5-G1R-dsRNA1 was diluted with normal saline before injection. The mice were weighed and an injection dose of 2 mg/kg was administered to the mice with abdominal depilation. The mice were injected subcutaneously by the following steps: grabbing and fixing the mice, wiping the skin between the two hind limbs with alcohol cotton balls, extracting the medicine according to the weight with the syringe, then gently piercing the skin with the needle inclined upward, and injecting slowly after running the needle subcutaneously about 1-2 cm along the skin. After the successful subcutaneous injection, the skin of mice at the injection site would bulge, and the needle would be pulled out smoothly in the opposite direction of needle entry to complete the injection. *In vivo* imaging was performed at different time points after injection to observe the distribution of Cy5.5-G1R-dsRNA1 after subcutaneous injection.

The results (**Fig. 5**) showed that obvious fluorescence could still be detected on Day 2 after the injection of Cy5.5-labeled Cy5.5-G1R-dsRNA1 adopting the present method, while the fluorescence could hardly be detected in the case of Cy5.5-labeled Cy5.5-Rs4 which did not adopt the enhancement method. It indicated that the dsRNA enhanced by the method still had good sustained release ability in animals.
dsRNA1_{:} (SEQ ID NO.11): 5'- UUGAAUGUAGAGAUGCGGUGG 
（SEQ ID NO.12）：5'- CCACCGCAUCUCUACAUUCAA
G1R：GGGUUGGGUUUGGGUUGGG
Rs4:

（SEQ ID NO.13）：5'-GCUGUAUCUUGUCUCCUCA UUGAAUGUAGAGAUGCGGUGG
GCUGUAUCUUGUCUCCUCA

（SEQ ID NO.14）：5'-UGAGGAGACAAGAUACAGC CCACCGCAUCUCUACAUUCAA
UGAGGAGACAAGAUACAGC

### Example 5: Results of evaluating the sustained release of antisense oligonucleotide (ASO) enhanced by the method of the present invention in animals.

In order to evaluate the ability of sustained release of ASO enhanced by the method of the present invention in animals, the ASO enhanced by the method was labeled with cy5.5. G1 sequences were added at both ends of CD35 sequence respectively, and Cy5.5 was used to form Cy5.5-G1-ASO1.

Cy5.5-G1-ASO1 was diluted with normal saline before injection. The mice were weighed and an injection dose of 2 mg/kg was administered to the mice with abdominal depilation. The mice were injected subcutaneously by the following steps: grabbing and fixing the mice, wiping the skin between the two hind limbs with alcohol cotton balls, extracting the medicine according to the weight with the syringe, then gently piercing the skin with the needle inclined upward, and injecting slowly after running the needle subcutaneously about 1-2 cm along the skin. After the successful subcutaneous injection, the skin of mice at the injection site would bulge, and the needle would be pulled out smoothly in the opposite direction of needle entry to complete the injection. *In vivo* imaging was performed at different time points after injection to observe the distribution of Cy5.5-G1-ASO1 after subcutaneous injection.

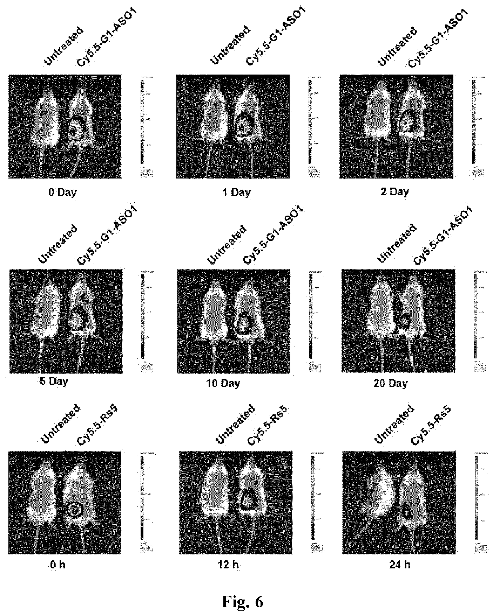

The results (Fig. 6) showed that the fluorescence could still be detected on Day 20 after the injection of Cy5.5-labeled Cy5.5-G1-ASO1 adopting the method, and the fluorescence on Day 20 was stronger than that of Cy5.5-labeled Cy5.5-Rs5 which did not adopt the enhancement method at 24 hours. It showed that ASO enhanced by the method still had good sustained release ability in animals.

Wherein the sequence information of said ASO is as follows:
ASO1 (SEQ ID NO.15)_{:} TTGAATGTAGAGATGCGGTGG
G1_{:} GGGTTGGGTTTGGGTTGGG

Rs5（SEQ ID NO.16）：GCTGTATCTTGTCTCCTCA TTGAATGTAGAGATGCGGTGG
GCTGTATCTTGTCTCCTCA

### Example 6. Results of evaluating different G-quadruplexes in enhancing the ability of sustained release of nucleic acids in animals.

Seven different G-quadruplex sequences were selected and respectively added to both ends of nucleic acid S1. The nucleic acid S1 without any sequence added was used as control. Cy5.5 was used for labeling, and the above sequences were injected into animals respectively to detect fluorescence.

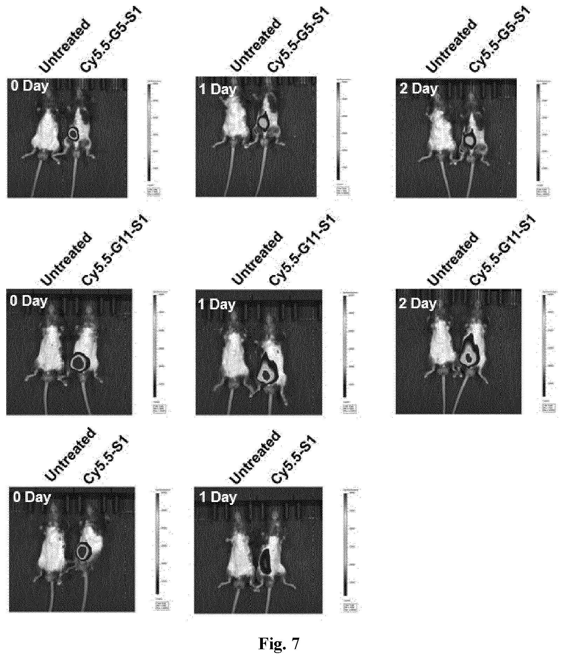

Some results (**Fig. 7**, results of G5 and G11) showed that different G-quadruplexes all had the ability of sustained release of nucleic acid. Wherein the number of continuous G in the G-quadruplex used was 2-4, the number of arbitrary bases was 1-6, the number of repetitions of continuous G was 4-16, and the number of repetitions of arbitrary bases in the middle interval was 3-15.

Wherein the sequence information of the G-quadruplex is as follows:
G0 (SEQ ID NO.17)_{:} GGGTAGGGCGGGTTGGG
G2 (SEQ ID NO.18): GGGTTTGGGTGGGTTΓGGG
G3 (SEQ ID NO.19): GGGTGGGTGGGTGGGT
G4 (SEQ ID NO.20): GGTGGTGGTGGTTGTGGTGGTGGTGG
G5 (SEQ ID NO.21): GGTGGTGGTGGTTGTTTGGTGGTGGTGG
G6 (SEQ ID NO.22): GTGGGGCATTGTGGGTGGGTGTGG
G7 (SEQ ID NO.23) : AGGGTTAGGGTTAGGGTTAGGG
G8 (SEQ ID NO.24): GGTTGGTGTGGTTGG
G9 (SEQ ID NO.25): GGGTAGGGCGGGGTTGGGG
G10 (SEQ ID NO.26)_{:} GGTGGTGGTGGTTGTGGTGGGTGGGTGGG
G11 (SEQ ID NO.27):
   GGTGGTGGTGGTTGTGGTGGTGGTGGTGGTGGTGGTGGTTGTGGTGGTGGTGG
S1 (SEQ ID NO.28)_{:} TTGAATGTAGAGATGCGGTGG

### Example 7: Results of evaluating sequences modified based on G1 in enhancing the ability of sustained release of nucleic acids in animals.

G1 sequence was selected to be modified in different ways. The modified sequences were added to both ends of the nucleic acid S1 respectively. The nucleic acid S1 without any sequence added was used as control. Cy5.5 was used for labeling, the sustained release ability of the modified sequences to the nucleic acid was evaluated, and the above sequences were injected into animals respectively to detect fluorescence.

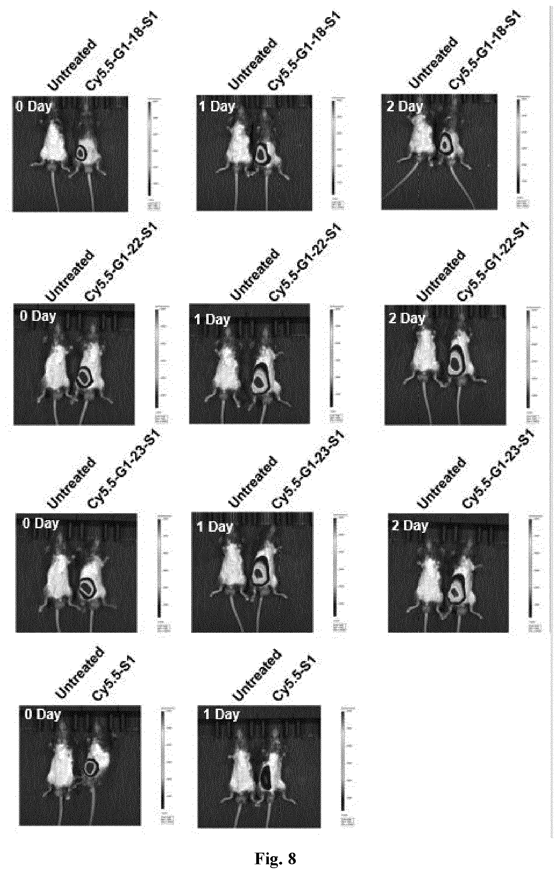

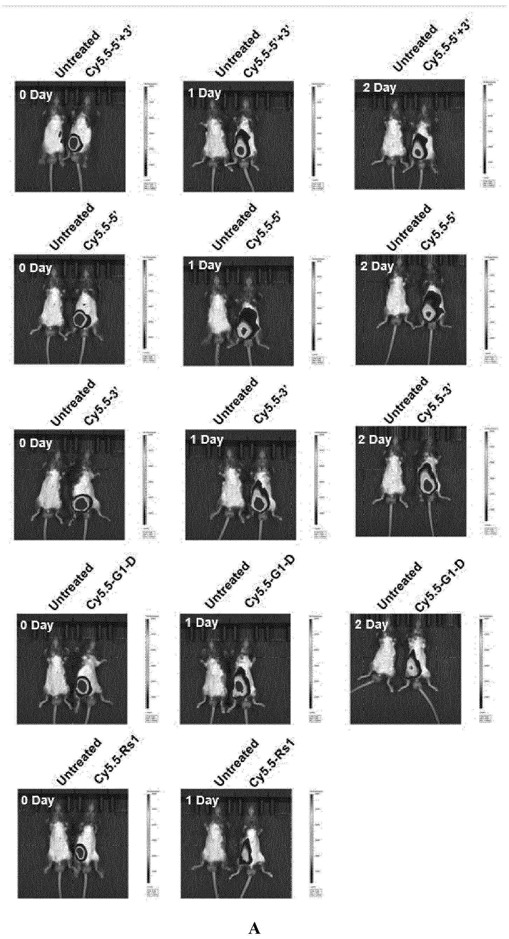

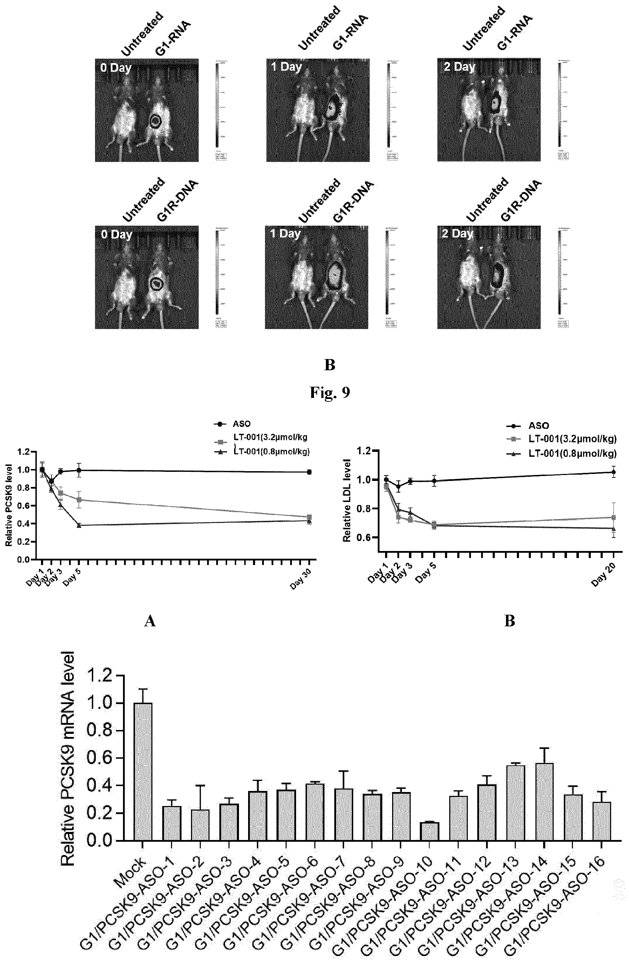

The results (**Fig. 8**, results of G1-18, G1-22 and G1-23) showed that different modifications all had the ability of sustained release to the nucleic acid, wherein the number of continuous G of the specific sequence used was 2-10, the number of arbitrary bases was 1-5, the number of repetitions of continuous G was 4-8, and the number of repetitions of arbitrary bases in the middle interval was 3-7.

Wherein the sequence information of the G1-based modified sequence was as follows:
G1-1 (SEQ ID NO.29): GGGAAGGGAGGGAAGGG
G1-2 (SEQ ID NO.30): GGGTTGGGTGGGTTGGG
G1-3 (SEQ ID NO.31): GGGCCGGGCGGGCCGGG
G1-4 (SEQ ID NO.32): GGGTTGGGTTGGGTTGGG
G1-5 (SEQ ID NO.33): GGGTGGGTTTTGGGTGGG
Gl-6 (SEQ ID NO.34): GGGTGGGTGGGTGGG
G1-7 (SEQ ID NO.35): GGGTGGGTTGGGTGGG
G1-8 (SEQ ID NO.36): GGGTGGGTTTGGGTGGG
G1-9 (SEQ ID NO.37): GGGTTTGGGTTTGGGTTTGGG
G1-10 (SEQ ID NO.38): GGGTTTTGGGTTTTGGGTTTTGGG
G1-11 (SEQ ID NO.39)_{:} GGGTTTTTGGGTTTTTGGGTTTTTGGG
G1-12 (SEQ ID NO.40)_{:} GGGGTTGGGGTTTGGGGTTGGGG
G1-13 (SEQ ID NO.41): GGGGGTTGGGGGTTTGGGGGTTGGGGG
G1-14 (SEQ ID NO.42): GGGGGGTTGGGGGGTTTGGGGGGTTGGGGGG
G1-15 (SEQ ID NO.43): GGGGGGGTTGGGGGGGTTTGGGGGGGTTGGGGGGG
G1-16 (SEQ ID NO.44): GGGGGGGGTTGGGGGGGGTTTGGGGGGGGTTGGGGGGGG
G1-17 (SEQ ID NO.45): GGGGGGGGGTTGGGGGGGGGTTTGGGGGGGGGTTGGGGGGGGG
G1-18 (SEQ ID NO.46): GGGGGGGGGGTTGGGGGGGGGGTTTGGGGGGGGGGTTGGGGGGGGGG
G1-19 (SEQ ID NO.47): GGGTAGGGCGGGTTGGGTAGGG
G1-20 (SEQ ID NO.48): GGGTAGGGCGGGTTGGGTAGGGCGGG
G1-21 (SEQ ID NO.49): GGGTAGGGCGGGTTGGGTAGGGCGGGTTGGG
G1-22 (SEQ ID NO.50): GGGTAGGGCGGGTTGGGGGGTAGGGCGGGTTGGG
G1-23 (SEQ ID NO.51): GGGTAGGGCGGGTTGGGTGGGTAGGGCGGGTTGGG
S1 (SEQ ID NO.28): TTGAATGTAGAGATGCGGTGG

### Example 8: Results of evaluating G1 at 5' end/and/or 3' end and G1 contained inside in enhancing the ability of sustained release of nucleic acids in animals.

In order to evaluate the ability of G1 at 5' end and/or 3' end to sustainedly release nucleic acid in animals, G1 was selected and added at 5' end, 3' end and both ends of nucleic acids having different lengths (S1, S2 and S3), wherein S1 was 21 nt, S2 was 8 nt and S3 was 13 nt. Cy5.5 was used for labeling and the above nucleic acids were injected into animals respectively to detect fluorescence.

The results (**Fig. 9A**) showed that G1 had sustained release ability to nucleic acids when it was added only at the 5' end (Cy5.5-5'), only at the 3' end (CY 5.5-3'), and at both ends (CY 5.5-5'+3').

In addition, the sustained release ability of G1 that was added within the nucleic acid sequence was evaluated. Two different sequences G1-In and G1-D were designed, in which G1-In was such a sequence that the G1 sequence appeared once inside the nucleic acid sequence, and G1-D was such a sequence that the G1 sequence was added at both ends of the target sequence and located inside the nucleic acid sequence. Cy5.5 was used for labeling, and the above sequences were injected into animals to detect fluorescence. The result (**Fig. 9A**) showed that G1 still had certain sustained release ability when it was inside the nucleic acid sequence.

Wherein:
S1 (SEQ ID NO.28)_{:} TTGAATGTAGAGATGCGGTGG
S2 (SEQID NO.52)_{:} AGACATTT
S3 (SEQ ID NO.53): ATTCTAGACATTT
G1-In (SEQID NO.54):
TTGAATGTAGAGATGCGGTGGGGGTTGGGTTTGGGTTGGGTTGAATGTAGAGATGCGGTGG
G1-D (SEQ ID NO.55): 
TTTGCCTTTAGGAGGGTTGGGTTTGGGTTGGGTTGAATGTAGAGATGCGGTGGGGGTTGGGTTTG
GGTTGGGTTCTAGACATTT
 wherein, the sequences underlined were G1.

### Example 9: Evaluation of the sustained release ability of chimera constructed by the method of the present invention in animals.

In order to evaluate the ability of chimera to sustainedly release nucleic acid in animals, two kinds of nucleic acids were selected, wherein G1 was selected for the sustained release of Sr (RNA) and G1R (RNA) was selected for the sustained release of S1 (DNA). The random sequence Rs1 was used as a control. Cy5.5 was used for labeling and the above nucleic acids were injected into animals respectively to detect fluorescence. The results (**Fig. 9B**) showed that G1 of different types (DNA/RNA) had sustained release ability for nucleic acids of different types (DNA/RNA), and it was not necessary to correspond completely.

The sequence information was as follows:
S1: TTGAATGTAGAGATGCGGTGG
Sr (SEQ ID NO.56)_{:} UUGAAUGUAGAGAUGCGGUGG
G1_{:} GGGTTGGGTTTGGGTTGGG
G1R_{:} GGGUUGGGUUUGGGUUGGG

### Example 10: Results of evaluating the ability of antisense oligonucleotide targeting PCSK9 sustainedly released by using the method of the present invention in knocking down PCSK9 and reducing LDL for a long time in animals.

In order to evaluate the effectiveness of antisense oligonucleotides enhanced by the method in animals, the invention designed an ASO sequence targeting PCSK9, and the method was used to enhance the sustained release ability of the ASO sequence. ASO without sustained release treatment was used as a negative control to compare the long-term effect of knocking down PCSK9 in animals by two different methods.

Before injection, the "ssDNA" needed for the experiment was diluted with normal saline. The mice were weighed and divided into three groups: ASO (0.8 µmol/kg), LT-001 (3.2 µmol/kg) and LT-001 (0.8 µmol/kg). 2 ml empty needle tubes and No.4.5 needles were used for injection. The diluted ssDNA saline solution was injected. Tail-cutting blood samples were taken every day in the first 3 days after injection, followed by tail-cutting blood samples on Day 5 and Day 30.

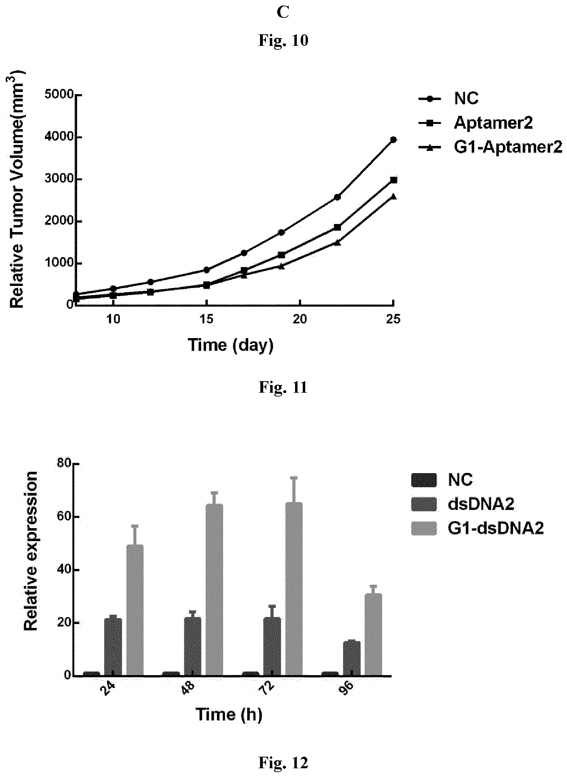

The contents of PCSK9 and LDL (low-density lipoprotein) in the plasma of mice were detected by ELISA. The effect of antisense oligonucleotide targeting PCSK9 sustainedly released by using the method of the present invention in knocking down PCSK9 for a long time in animals was investigated. The results (**Figs. 10A and 10B**) showed that the values of PCSK9 and LDL in mice injected with LT-001 were significantly lower than those injected with ASO without sustained release treatment, and they were still effective on Day 30. It showed that the method of the present invention enhanced the sustained release ability of antisense oligonucleotide targeting PCSK9 in animals, knocking down PCSK9 and reducing LDL content for a long time.

The sequence information was as follows: 
LT-001（SEQ ID NO.57）：GGGTTGGGTTTGGGTTGGG GTCTGTGGAAGCG
GGGTTGGGTTTGGGTTGGG
 ASO (SEQ ID NO.58): GTCTGTGGAAGCG

Furthermore, 16 specific ASOs were designed according to PCSK9 mRNA, and G1 was added at both ends of ASOs (G1/PCSK9-ASO). G1/PCSK9-ASO was transfected into hepatocellular carcinoma cell HCC97H with lipotamine2000 liposome at a dose of 20 pmol. Cells were collected at 48 hours after transfection, the total RNA of cells was extracted with TRIZOL reagent, and the knock-down effect of PCSK9 mRNA was detected by RT-qPCR. The results showed that 16 G1/PCSK9-ASOs could effectively knock down PCSK9 mRNA (**Fig. 10C**).

The sequence information was as follows:
PCSK9-ASO-1 (SEQ ID NO.83) :
AGCCACGTGGGCAGCAGCCTGTGA G1/PCSK9-ASO-1 (SEQID NO.59) :
GGGTTGGGTTTGGGTTGGGAGCCACGTGGGCAGCAGCCTGTGAGGGTTGGGTTTGGGTTGGG PCSK9-ASO-2 (SEQ ID NO.84) :
TTCCACGTGGGCAGCAGCCTGTTT G1/PCSK9-ASO-2 (SEQID NO.60) :
GGGTTGGGTTTGGGTTGGGTTCCACGTGGGCAGCAGCCTGTTTGGGTTGGGTTTGGGTTGGG PCSK9-ASO-3 (SEQ ID NO.85) :
CGTAGACACCCTCACCCCCA G1/PCSK9-ASO-3 (SEQID NO.61) :
GGGTTGGGTTTGGGTTGGGCGTAGACACCCTCACCCCCAGGGTTGGGTTTGGGTTGGG PCSK9-ASO-4 (SEQ ID NO.86) :
TTTAGACACCCTCACCCCTT G1/PCSK9-ASO-4 (SEQID NO.62) :
GGGTTGGGTTTGGGTTGGGTTTAGACACCCTCACCCCTTGGGTTGGGTTTGGGTTGGG PCSK9-ASO-5 (SEQ ID NO.87) :
TTTAGACACCCTCACCCCCAATT G1/PCSK9-ASO-5 (SEQID NO.63) :
GGGTTGGGTTTGGGTTGGGTTTAGACACCCTCACCCCCAATTGGGTTGGGTTTGGGTTGGG PCSK9-ASO-6 (SEQ ID NO.88) :
CGTAGACACCCTCACCCCCAAAA G1/PCSK9-ASO-6 (SEQ ID NO.64) :
GGGTTGGGTTTGGGTTGGGCGTAGACACCCTCACCCCCAAAAGGGTTGGGTTTGGGTTGGG PCSK9-ASO-7 (SEQ ID NO.89) :
TTCATCCCGGCCGCTGACCTT G1/PCSK9-ASO-7 (SEQID NO.65) :
GGGTTGGGTTTGGGTTGGGTTCATCCCGGCCGCTGACCTTGGGTTGGGTTTGGGTTGGG PCSK9-ASO-8 (SEQ ID NO.90) :
TTTCCCCAAAGTCCCCTT G1/PCSK9-ASO-8 (SEQ ID NO.66) :
GGGTTGGGTTTGGGTTGGGTTTCCCCAAAGTCCCCTTGGGTTGGGTTTGGGTTGGG PCSK9-ASO-9 (SEQ ID NO.91) :
TTCCACGTGGGCAGCAGCCTGTT G1/PCSK9-ASO-9 (SEQ ID NO.67) :
GGGTTGGGTTTGGGTTGGGTTCCACGTGGGCAGCAGCCTGTTGGGTTGGGTTTGGGTTGGG PCSK9-ASO-10 (SEQ ID NO.92) :
TTGCCACGTGGGCAGCAGCCTGTT G1/PCSK9-ASO-10 (SEQID NO.68) :
GGGTTGGGTTTGGGTTGGGTTGCCACGTGGGCAGCAGCCTGTTGGGTTGGGTTTGGGTTGGG PCSK9-ASO-11 (SEQID NO.93) :
TTTCAGGGAACCAGGCTT G1/PCSK9-ASO-11 (SEQID NO.69) :
GGGTTGGGTTTGGGTTGGGTTTCAGGGAACCAGGCTTGGGTTGGGTTTGGGTTGGG PCSK9-ASO-12 (SEQID NO.94) :
TTTCCTCAGGGAACCATT G1/PCSK9-ASO-12 (SEQID NO.70) :
GGGTTGGGTTTGGGTTGGGTTTCCTCAGGGAACCATTGGGTTGGGTTTGGGTTGGG PCSK9-ASO-13 (SEQID NO.95) :
TTGCTCCGGCAGCAGATTT G1/PCSK9-ASO-13 (SEQID NO.71) :
GGGTTGGGTTTGGGTTGGGTTGCTCCGGCAGCAGATTTGGGTTGGGTTTGGGTTGGG PCSK9-ASO-14 (SEQID NO.96) :
TTGGGATGCTCTGGGCTT Gl/PCSK9-ASO-14 (SEQID NO.72) :
GGGTTGGGTTTGGGTTGGGTTGGGATGCTCTGGGCTTGGGTTGGGTTTGGGTTGGG PCSK9-ASO-15 (SEQID NO.97) :
TTGCCTGTCTGTGGAATT G1/PCSK9-ASO-15 (SEQID NO.73) :
GGGTTGGGTTTGGGTTGGGTTGCCTGTCTGTGGAATTGGGTTGGGTTTGGGTTGGG PCSK9-ASO-16 (SEQID NO.98) :
TTCTGGTCCTCAGGGAACCAGGCCTT G1/PCSK9-ASO-16 (SEQ ID NO.74) :
GGGTTGGGTTTGGGTTGGGTTCTGGTCCTCAGGGAACCAGGCCTTGGGTTGGGTTTGGGTTGGG

### Example 11: Results of evaluating the effect of the aptamer sustainedly released by using the method of the present invention on long-term inhibition of protein in animals.

In order to evaluate the ability of the method to sustainedly release nucleic acid aptamers to inhibit protein activity for a long time, aptamer was selected to test the inhibitory protein activity and was injected into mice to evaluate the protein activity. Compared with the control (Aptamer2), the protein activity in the animals injected with G1-Aptamer2 that G1 was added at both ends of aptamer was significantly lower than that of Aptamer2, which indicated that this method enhanced the sustained release ability of the aptamer, so that the activity of protein was inhibited for a long time.

Aptamer of FGL-1, Aptamer that G1 was added at one end (G1 Aptamer2) and its control (Aptamer2) was screened.

Six days after inoculation with tumor cells, the tumor volume of mice was about 100 mm³. At this time, mice were randomly divided into two groups, with 6 mice in each group. Mice were weighed and given subcutaneous injection of G1 Aptamer2 and Aptamer2 at an injection dose of 15 mg/kg, respectively, with normal saline injection as the blank control (NC).

The changes of tumor size and weight in mice were monitored every 2 or 3 days. The long diameter (L) and short diameter (S) of mice tumor were measured by digital vernier caliper, and the average value of three readings was recorded during each measurement, and the tumor volume of mice was calculated according to the formula: V =L × S²/2.

The results (**Fig. 11**) showed that the anti-tumor effect of animals injected with G1 Aptamer2 was better than that of Aptamer2, which showed that the sustained release ability of nucleic acid aptamer was enhanced by this method, so that the activity of protein was inhibited for a long time.

The sequence information was as follows:
Aptamer2 (SEQ ID NO.75): CGGGTTTGGCTCCGGGTCTGGCGCGACCTCATCCTACTCA
G1-Aptamer2 (SEQ ID NO.76);
GGGTTGGGTTTGGGTTGGGTTTCGGGTTTGGCTCCGGGTCTGGCGCGACCTCATCCTACTCA

### Example 12: Results of evaluating the ability of the method of the present invention to sustainedly release DNA in transcribing RNA for a long time in animals.

In order to evaluate the ability of the method of the present invention to enhance the sustained release ability of dsDNA, the DNA of fluorescent protein was selected for transcriptional ability testing. The DNA was injected into mice, and the mRNA expression of fluorescent protein was analyzed by qPCR method. Compared with the control (dsDNA2), the mRNA content of fluorescent protein in animals injected with G1-dsDNA2 that G1 was added at both ends of the dsDNA was significantly higher than that of animals injected with dsDNA1, which indicated that the sustained release ability of dsDNA was enhanced by this method, so that the dsDNA was transcribed for a long time.

The dsDNA2 with GFP was selected for transcriptional ability testing. G1 was added at both ends of the dsDNA2 sequence (G1-dsDNA2), and the mRNA expression of fluorescent protein was analyzed by qPCR method.

Before injection, dsDNA2 and G1-dsDNA2 were diluted with normal saline. The mice were weighed and administered subcutaneously at an injection dose of 4 mg/kg. Plasma samples were taken at 24/48/72/96 hours after injection for fluorescence quantitative detection. Normal saline was injected as a blank control (NC).

The results (**Fig. 12**) showed that the mRNA content of the fluorescent protein in animals injected with G1-dsDNA2 was significantly higher than that of animals injected with dsDNA2, indicating that the sustained release ability of DSDNA was enhanced by this method, so that dsDNA was transcribed for a long time.

The sequence information was as follows (the underlined part was G1 sequence):
dsDNA2 (SEQ ID NO.77): 
GACATTGATTATTGACTAGTTATTAATAGTAATCAATTACGGGGTCATTAGTTCATAGCCCATATATG
GAGTTCCGCGTTACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACGTCAATGGGTGGA
GTATTTACGGTAAACTGCCCACTTGGCAGTACATCAAGTGTATCATATGCCAAGTACGCCCCCTATTGA
CGTCAATGACGGTAAATGGCCCGCCTGGCATTATGCCCAGTACATGACCTTATGGGACTTTCCTACTTG
GCAGTACATCTACGTATTAGTCATCGCTATTACCATGGTGATGCGGTTTTGGCAGTACATCAATGGGCGT
GGATAGCGGTTTGACTCACGGGGATTTCCAAGTCTCCACCCCATTGACGTCAATGGGAGTTTGTTTTG
GCACCAAAATCAACGGGACTTTCCAAAATGTCGTAACAACTCCGCCCCATTGACGCAAATGGGCGGT
AGGCGTGTACGGTGGGAGGTCTATATAAGCAGAGCTCTCTGGCTAACTAGAGAACCCACTGCTTACTG
GCTTATCGAAATTAATACGACTCACTATAGGGAGACCCAAGCTGGCTAGTTAAGCTTCACCATGGTGA
GCAAGGGCGAGGAGCTGTTCACCGGGGTGGTGCCCATCCTGGTCGAGCTGGACGGCGACGTAAACG
GCCACAAGTTCAGCGTGTCCGGCGAGGGCGAGGGCGATGCCACCTACGGCAAGCTGACCCTGAAGT
TCATCTGCACCACCGGCAAGCTGCCCGTGCCCTGGCCCACCCTCGTGACCACCCTGACCTACGGCGT
GCAGTGCTTCAGCCGCTACCCCGACCACATGAAGCAGCACGACTTCTTCAAGTCCGCCATGCCCGAA
GGCTACGTCCAGGAGCGCACCATCTTCTTCAAGGACGACGGCAACTACAAGACCCGCGCCGAGGTG
AAGTTCGAGGGCGACACCCTGGTGAACCGCATCGAGCTGAAGGGCATCGACTTCAAGGAGGACGGC
AACATCCTGGGGCACAAGCTGGAGTACAACTACAACAGCCACAACGTCTATATCATGGCCGACAAGC
AGAAGAACGGCATCAAGGTGAACTTCAAGATCCGCCACAACATCGAGGACGGCAGCGTGCAGCTCG
CCGACCACTACCAGCAGAACACCCCCATCGGCGACGGCCCCGTGCTGCTGCCCGACAACCACTACCT
GAGCACCCAGTCCAAGCTGAGCAAAGACCCCAACGAGAAGCGCGATCACATGGTCCTGCTGGAGTT
CGTGACCGCCGCCGGGATCACTCTCGGCATGGACGAGCTGTACAAGTAATAAGGATCCAGTACCC
TTCACCAAAGCAAAAATGTGATCTTGCTTGTAAATACAATTTTGAGAGGTTAATAAATTACAAGTAGTG
CTATTTTTGTATTTAGGTTAGCTATTTAGCTTTACGTTCCAGGATGCCTAGTGGCAGCCCCACAATATCC
AGGAAGCCCTCTCTGCGGTTTTTCAGATTAGGTAGTCGAAAAACCTAAGAAATTTACCTGCT
G1-dsDNA2 (SEQ ID NO.78):

GGGTTGGGTTTGGGTTGGGGACATTGATTATTGACTAGTTATTAATAGTAATCAATTACGGGGTCAT
TAGTTCATAGCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAGGGACTTTCC
ATTGACGTCAATGGGTGGAGTATTTACGGTAAACTGCCCACTTGGCAGTACATCAAGTGTATCATATGC
CAAGTACGCCCCCTATTGACGTCAATGACGGTAAATGGCCCGCCTGGCATTATGCCCAGTACATGACCT
TATGGGACTTTCCTACTTGGCAGTACATCTACGTATTAGTCATCGCTATTACCATGGTGATGCGGTTTTG
GCAGTACATCAATGGGCGTGGATAGCGGTTTGACTCACGGGGATTTCCAAGTCTCCACCCCATTGACG
TCAATGGGAGTTTGTTTTGGCACCAAAATCAACGGGACTTTCCAAAATGTCGTAACAACTCCGCCCCA
TTGACGCAAATGGGCGGTAGGCGTGTACGGTGGGAGGTCTATATAAGCAGAGCTCTCTGGCTAACTAG
AGAACCCACTGCTTACTGGCTTATCGAAATTAATACGACTCACTATAGGGAGACCCAAGCTGGCTAGT
TAAGCTTCACCATGGTGAGCAAGGGCGAGGAGCTGTTCACCGGGGTGGTGCCCATCCTGGTCGAGCT
GGACGGCGACGTAAACGGCCACAAGTTCAGCGTGTCCGGCGAGGGCGAGGGCGATGCCACCTACGG
CAAGCTGACCCTGAAGTTCATCTGCACCACCGGCAAGCTGCCCGTGCCCTGGCCCACCCTCGTGACC
ACCCTGACCTACGGCGTGCAGTGCTTCAGCCGCTACCCCGACCACATGAAGCAGCACGACTTCTTCA
AGTCCGCCATGCCCGAAGGCTACGTCCAGGAGCGCACCATCTTCTTCAAGGACGACGGCAACTACAA
GACCCGCGCCGAGGTGAAGTTCGAGGGCGACACCCTGGTGAACCGCATCGAGCTGAAGGGCATCGA
CTTCAAGGAGGACGGCAACATCCTGGGGCACAAGCTGGAGTACAACTACAACAGCCACAACGTCTAT
ATCATGGCCGACAAGCAGAAGAACGGCATCAAGGTGAACTTCAAGATCCGCCACAACATCGAGGAC
GGCAGCGTGCAGCTCGCCGACCACTACCAGCAGAACACCCCCATCGGCGACGGCCCCGTGCTGCTGC
CCGACAACCACTACCTGAGCACCCAGTCCAAGCTGAGCAAAGACCCCAACGAGAAGCGCGATCACA
TGGTCCTGCTGGAGTTCGTGACCGCCGCCGGGATCACTCTCGGCATGGACGAGCTGTACAAGTAATAA
TAAGGATCCAGTACCCTTCACCAAAGCAAAAATGTGATCTTGCTTGTAAATACAATTTTGAGAGGTTAA
TAAATTACAAGTAGTGCTATTTTTGTATTTAGGTTAGCTATTTAGCTTTACGTTCCAGGATGCCTAGTGG
CAGCCCCACAATATCCAGGAAGCCCTCTCTGCGGTTTTTCAGATTAGGTAGTCGAAAAACCTAAGAAA
TTTACCTGCTGGGTTGGGTTTGGGTTGGG

### Example 13: Results of evaluating the ability of the method of the present invention in sustainedly release DNA to translate proteins in animals for a long time.

In order to evaluate the ability of the method of the present invention in enhancing the sustained release ability of RNA, the RNA of fluorescent protein was selected for sustained release ability testing. The RNA transcribed in vitro was injected into mice, and the fluorescence intensity of fluorescent protein was measured. Compared with the control (mRNA1), the eGFP fluorescence maintenance time in animals injected with G1-mRNA1 that G1 was added at both ends of the RNA was longer than that of animals injected with mRNA 1, which indicated that the sustained release ability of mRNA was enhanced by this method, so that the RNA was translated for a long time.

Firefly luciferase was selected as a marker gene (mRNA1) for mRNA sustained release ability testing. G1R was added at both ends of mRNA1 to form G1-mRNA1, and the plasmid with firefly luciferase gene was used as a positive control (PC).

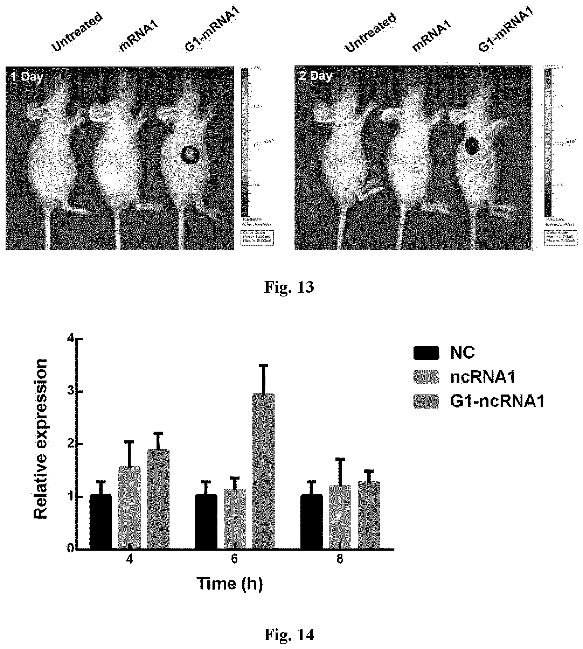

After the mRNA (or plasmid) transcribed *in vitro* was diluted with sterile DPBS, nude mice were weighed and injected subcutaneously according to the injection dose of 5 mg/kg. After the successful subcutaneous injection, the skin of mice would bulge. Before *in vivo* imaging observation at different time points after injection, the substrate D-Luciferin (Perkin Elmer, catalog number: 770504) was injected into the tail vein at a dose of 150 mg/kg. After 5 minutes, the mice were treated with gas anesthesia (3% isoflurane) and put into IVIS small animal *in vivo* imaging system for imaging. Imaging was taken 20 minutes after substrate injection, and the results showed (**Fig. 13**) that the fluorescence maintenance time of animals injected with G1-mRNA1 could reach 48 hours, indicating that this method enhanced the sustained release ability of mRNA, so that translating the protein for a long time.

The sequence information was as follows:
mRNA1 (SEQ ID NO.79): 
UUAAAACAGCCUGUGGGUUGAUCCCACCCACAGGCCCAUUGGGCGCUAGCACUCUGGUAUCA
CGGUACCUUUGUGCGCCUGUUUUAUACCCCCUCCCCCAACUGUAACUUAGAAGUAACACACACCG
AUCAACAGUCAGCGUGGCACACCAGCCACGUUUUGAUCAAGCACUUCUGUUACCCCGGACUGAGU
AUCAAUAGACUGCUCACGCGGUUGAAGGAGAAAGCGUUCGUUAUCCGGCCAACUACUUCGAAAA
ACCUAGUAACACCGUGGAAGUUGCAGAGUGUUUCGCUCAGCACUACCCCAGUGUAGAUCAGGUC
GAUGAGUCACCGCAUUCCCCACGGGCGACCGUGGCGGUGGCUGCGUUGGCGGCCUGCCCAUGGGG
AAACCCAUGGGACGCUCUAAUACAGACAUGGUGCGAAGAGUCUAUUGAGCUAGUUGGUAGUCCU
CCGGCCCCUGAAUGCGGCUAAUCCUAACUGCGGAGCACACACCCUCAAGCCAGAGGGCAGUGUGU
CGUAACGGGCAACUCUGCAGCGGAACCGACUACUUUGGGUGUCCGUGUUUCAUUUUAUUCCUAU
ACUGGCUGCUUAUGGUGACAAUUGAGAGAUCGUUACCAUAUAGCUAUUGGAUUGGCCAUCCGGU
GACUAAUAGAGCUAUUAUAUAUCCCUUUGUUGGGUUUAUACCACUUAGCUUGAAAGAGGUUAAA
ACAUUACAAUUCAUUGUUAAGUUGAAUACAGCAAAAUGGCCGAUGCUAAGAACAUUAAGAAGGG
CCCUGCUCCCUUCUACCCUCUGGAGGAUGGCACCGCUGGCGAGCAGCUGCACAAGGCCAUGAAGA
GGUAUGCCCUGGUGCCUGGCACCAUUGCCUUCACCGAUGCCCACAUUGAGGUGGACAUCACCUAU
GCCGAGUACUUCGAGAUGUCUGUGCGCCUGGCCGAGGCCAUGAAGAGGUACGGCCUGAACACCA
ACCACCGCAUCGUGGUGUGCUCUGAGAACUCUCUGCAGUUCUUCAUGCCAGUGCUGGGCGCCCUG
UUCAUCGGAGUGGCCGUGGCCCCUGCUAACGACAUUUACAACGAGCGCGAGCUGCUGAACAGCAU
GGGCAUUUCUCAGCCUACCGUGGUGUUCGUGUCUAAGAAGGGCCUGCAGAAGAUCCUGAACGUG
CAGAAGAAGCUGCCUAUCAUCCAGAAGAUCAUCAUCAUGGACUCUAAGACCGACUACCAGGGCU
UCCAGAGCAUGUACACAUUCGUGACAUCUCAUCUGCCUCCUGGCUUCAACGAGUACGACUUCGUG
CCAGAGUCUUUCGACAGGGACAAAACCAUUGCCCUGAUCAUGAACAGCUCUGGGUCUACCGGCCU
GCCUAAGGGCGUGGCCCUGCCUCAUCGCACCGCCUGUGUGCGCUUCUCUCACGCCCGCGACCCUA
UUUUCGGCAACCAGAUCAUCCCCGACACCGCUAUUCUGAGCGUGGUGCCAUUCCACCACGGCUUC
GGCAUGUUCACCACCCUGGGCUACCUGAUUUGCGGCUUUCGGGUGGUGCUGAUGUACCGCUUCG
AGGAGGAGCUGUUCCUGCGCAGCCUGCAAGACUACAAAAUUCAGUCUGCCCUGCUGGUGCCAACC
CUGUUCAGCUUCUUCGCUAAGAGCACCCUGAUCGACAAGUACGACCUGUCUAACCUGCACGAGAU
UGCCUCUGGCGGCGCCCCACUGUCUAAGGAGGUGGGCGAAGCCGUGGCCAAGCGCUUUCAUCUGC
CAGGCAUCCGCCAGGGCUACGGCCUGACCGAGACAACCAGCGCCAUUCUGAUUACCCCAGAGGGC
GACGACAAGCCUGGCGCCGUGGGCAAGGUGGUGCCAUUCUUCGAGGCCAAGGUGGUGGACCUGG
ACACCGGCAAGACCCUGGGAGUGAACCAGCGCGGCGAGCUGUGUGUGCGCGGCCCUAUGAUUAU
GUCCGGCUACGUGAAUAACCCUGAGGCCACAAACGCCCUGAUCGACAAGGACGGCUGGCUGCACU
CUGGCGACAUUGCCUACUGGGACGAGGACGAGCACUUCUUCAUCGUGGACCGCCUGAAGUCUCUG
AUCAAGUACAAGGGCUACCAGGUGGCCCCAGCCGAGCUGGAGUCUAUCCUGCUGCAGCACCCUAA
CAUUUUCGACGCCGGAGUGGCCGGCCUGCCCGACGACGAUGCCGGCGAGCUGCCUGCCGCCGUCG
UCGUGCUGGAACACGGCAAGACCAUGACCGAGAAGGAGAUCGUGGACUAUGUGGCCAGCCAGGU
GACAACCGCCAAGAAGCUGCGCGGCGGAGUGGUGUUCGUGGACGAGGUGCCCAAGGGCCUGACC
GGCAAGCUGGACGCCCGCAAGAUCCGCGAGAUCCUGAUCAAGGCUAAGAAAGGCGGCAAGAUCG
CCGUGUAA
G1-mRNA1 (SEQ ID NO.80):

GGGUUGGGUUUGGGUUGGGUUAAAACAGCCUGUGGGUUGAUCCCACCCACAGGCCCAUUGG
GCGCUAGCACUCUGGUAUCACGGUACCUUUGUGCGCCUGUUUUAUACCCCCUCCCCCAACUGUAA
CUUAGAAGUAACACACACCGAUCAACAGUCAGCGUGGCACACCAGCCACGUUUUGAUCAAGCACU
UCUGUUACCCCGGACUGAGUAUCAAUAGACUGCUCACGCGGUUGAAGGAGAAAGCGUUCGUUAU
CCGGCCAACUACUUCGAAAAACCUAGUAACACCGUGGAAGUUGCAGAGUGUUUCGCUCAGCACU
ACCCCAGUGUAGAUCAGGUCGAUGAGUCACCGCAUUCCCCACGGGCGACCGUGGCGGUGGCUGCG
UUGGCGGCCUGCCCAUGGGGAAACCCAUGGGACGCUCUAAUACAGACAUGGUGCGAAGAGUCUA
UUGAGCUAGUUGGUAGUCCUCCGGCCCCUGAAUGCGGCUAAUCCUAACUGCGGAGCACACACCCU
CAAGCCAGAGGGCAGUGUGUCGUAACGGGCAACUCUGCAGCGGAACCGACUACUUUGGGUGUCC
GUGUUUCAUUUUAUUCCUAUACUGGCUGCUUAUGGUGACAAUUGAGAGAUCGUUACCAUAUAGC
UAUUGGAUUGGCCAUCCGGUGACUAAUAGAGCUAUUAUAUAUCCCUUUGUUGGGUUUAUACCAC
UUAGCUUGAAAGAGGUUAAAACAUUACAAUUCAUUGUUAAGUUGAAUACAGCAAAAUGGCCGAU
GCUAAGAACAUUAAGAAGGGCCCUGCUCCCUUCUACCCUCUGGAGGAUGGCACCGCUGGCGAGCA
GCUGCACAAGGCCAUGAAGAGGUAUGCCCUGGUGCCUGGCACCAUUGCCUUCACCGAUGCCCACA
UUGAGGUGGACAUCACCUAUGCCGAGUACUUCGAGAUGUCUGUGCGCCUGGCCGAGGCCAUGAA
GAGGUACGGCCUGAACACCAACCACCGCAUCGUGGUGUGCUCUGAGAACUCUCUGCAGUUCUUCA
UGCCAGUGCUGGGCGCCCUGUUCAUCGGAGUGGCCGUGGCCCCUGCUAACGACAUUUACAACGAG
CGCGAGCUGCUGAACAGCAUGGGCAUUUCUCAGCCUACCGUGGUGUUCGUGUCUAAGAAGGGCC
UGCAGAAGAUCCUGAACGUGCAGAAGAAGCUGCCUAUCAUCCAGAAGAUCAUCAUCAUGGACUC
UAAGACCGACUACCAGGGCUUCCAGAGCAUGUACACAUUCGUGACAUCUCAUCUGCCUCCUGGCU
UCAACGAGUACGACUUCGUGCCAGAGUCUUUCGACAGGGACAAAACCAUUGCCCUGAUCAUGAA
CAGCUCUGGGUCUACCGGCCUGCCUAAGGGCGUGGCCCUGCCUCAUCGCACCGCCUGUGUGCGCU
UCUCUCACGCCCGCGACCCUAUUUUCGGCAACCAGAUCAUCCCCGACACCGCUAUUCUGAGCGUG
GUGCCAUUCCACCACGGCUUCGGCAUGUUCACCACCCUGGGCUACCUGAUUUGCGGCUUUCGGGU
GGUGCUGAUGUACCGCUUCGAGGAGGAGCUGUUCCUGCGCAGCCUGCAAGACUACAAAAUUCAG
UCUGCCCUGCUGGUGCCAACCCUGUUCAGCUUCUUCGCUAAGAGCACCCUGAUCGACAAGUACGA
CCUGUCUAACCUGCACGAGAUUGCCUCUGGCGGCGCCCCACUGUCUAAGGAGGUGGGCGAAGCCG
UGGCCAAGCGCUUUCAUCUGCCAGGCAUCCGCCAGGGCUACGGCCUGACCGAGACAACCAGCGCC
AUUCUGAUUACCCCAGAGGGCGACGACAAGCCUGGCGCCGUGGGCAAGGUGGUGCCAUUCUUCG
AGGCCAAGGUGGUGGACCUGGACACCGGCAAGACCCUGGGAGUGAACCAGCGCGGCGAGCUGUG
UGUGCGCGGCCCUAUGAUUAUGUCCGGCUACGUGAAUAACCCUGAGGCCACAAACGCCCUGAUCG
ACAAGGACGGCUGGCUGCACUCUGGCGACAUUGCCUACUGGGACGAGGACGAGCACUUCUUCAUC
GUGGACCGCCUGAAGUCUCUGAUCAAGUACAAGGGCUACCAGGUGGCCCCAGCCGAGCUGGAGUC
UAUCCUGCUGCAGCACCCUAACAUUUUCGACGCCGGAGUGGCCGGCCUGCCCGACGACGAUGCCG
GCGAGCUGCCUGCCGCCGUCGUCGUGCUGGAACACGGCAAGACCAUGACCGAGAAGGAGAUCGUG
GACUAUGUGGCCAGCCAGGUGACAACCGCCAAGAAGCUGCGCGGCGGAGUGGUGUUCGUGGACG
AGGUGCCCAAGGGCCUGACCGGCAAGCUGGACGCCCGCAAGAUCCGCGAGAUCCUGAUCAAGGCU
AAGAAAGGCGGCAAGAUCGCCGUGUAAGGGUUGGGUUUGGGUUGGG

### Example 14: Evaluation of the ability of the method of the present invention in slowly releasing ncRNA to realize the long-term effect of ncRNA in animals.

In order to evaluate the ability of the method of the present invention in enhancing the sustained release ability of ncRNA, ncRNA (which had the ability to regulate the target) was selected for the sustained release ability testing and injected into mice. Compared with the control (ncRNA1), the animals injected with G1-ncRNA1 that G1 was added at both ends of the ncRNA1 regulated the target for a significantly longer time than the animal injected with ncRNA1, which showed that this method enhanced the sustained release ability of ncRNA and prolonged the effect of ncRNA.

BGLT3 (ncRNA1) was selected for the sustained release ability testing. It had been pointed out that BGL3 positively regulated the expression of its upstream gene HBG1 (Ivaldims.etc.blood.). G1 was added at both ends of ncRNA1 to form G1-ncRNA1. The qPCR method was used to analyze the expression of HGB1.

Before injection, ncRNA was diluted with normal saline. 2-week-old BAKB/c mice were weighed and injected subcutaneously at an injection dose of 4 mg/kg, with normal saline as a blank control (NC). Liver RNA was extracted at 4/6/8 hours after injection, respectively. The expression of HGB1 was detected by qPCR.

The results (**Fig. 14**) showed that the animals injected with G1-ncRNA1 regulated the HGB1 for a significantly longer time than the animal injected with ncRNA1, which indicated that this method enhanced the sustained release ability of ncRNA and prolonged the effect of ncRNA.

The sequence information was as follows:
ncRNA1 (SEQ ID NO.81): 
UUAGCAGUAACUGCUGAAUUCCUGGUUGGCUGAUGGAAAGAUGGGGCAGCUGUUCACUGGU
ACGCAGGGUUUUAGAUGUAUGUACCUAAGGAUAUGAGGUAUGGCAAUGAACAGAAAUUCUUUUG
GGAAUGAGUUUUAGGGCCAUUAAAGGACAUGACCUGAAGUUUCCUCUGAGGCCAGUCCCCACAA
CUCAAUAUAAAUGUGUUUCCUGCAUAUAGUCAAAGUUGCCACUUCUUUUUCUUCAUAUCAUCGA
UCUCUGCUCUUAAAGAUAAUCUUGGUUUUGCCUCAAACUGUUUGUCACUACAAACUUUCCCCAU
GUUCCUAAGUAAAACAGGUAACUGCCUCUCAACUAUAUCAAGUAGACUAAAAUAUUGUGUCUCU
AAUAUCAGAAAUUCAGCUUUAAUAUAUUGGGUUUAACUCUUUGAAAUUUAGAGUCUCCUUGAAA
UACACAUGGGGGUGAUUUCCUAAACUUUAUUUCUUGUAAGGAUUUAUCUCAGGGGUAACACACA
AACCAGCAUCCUGAACCUCUAAGUAUGAGGACAGUAAGCCUUAAGAAUAUAAAAUAAACUGUUC
UUCUCUCUGCCGGUGGAAGUGUGCCCUGUCUAUUCCUGAAAUUGCUUGUUUGAGACGCAUGAGA
CGUGCAGCACAUGAGACACGUGCAGCAGCCUGUGGAAUAUUGUCAGUGAAGAAUGUCUUUGCCU
GAUUAGAUAUAAAGACAAGUUAAACACAGCAUUAGACUAUAGAUCAAGCCUGUGCCAGACACAA
AUGACCUAAUGCCCAGCACGGGCCACGGAAUCUCCUAUCCUCUUGCUUGAACAGAGCAGCACACU
UCUCCCCCAACACUAUUAGAUGUUCUGGCAUAAUUUUGUAGAUAUGUAGGAUUUGACAUGGACU
AUUGUUCAAUGAUUCAGAGGAAAUCUCCUUUGUUCAGAUAAGUACACUGACUACUAAAUGGAUU
AAAAAACACAGUAAUAAAACCCAGUUUUCCCCUUAAAAAAAAAAAAAAAAAAAAAAAAAA
G1-ncRNA1 (SEQ ID NO.82):

GGGUUGGGUUUGGGUUGGGUUAGCAGUAACUGCUGAAUUCCUGGUUGGCUGAUGGAAAGAU
GGGGCAGCUGUUCACUGGUACGCAGGGUUUUAGAUGUAUGUACCUAAGGAUAUGAGGUAUGGCA
AUGAACAGAAAUUCUUUUGGGAAUGAGUUUUAGGGCCAUUAAAGGACAUGACCUGAAGUUUCCU
CUGAGGCCAGUCCCCACAACUCAAUAUAAAUGUGUUUCCUGCAUAUAGUCAAAGUUGCCACUUC
UUUUUCUUCAUAUCAUCGAUCUCUGCUCUUAAAGAUAAUCUUGGUUUUGCCUCAAACUGUUUGU
CACUACAAACUUUCCCCAUGUUCCUAAGUAAAACAGGUAACUGCCUCUCAACUAUAUCAAGUAG
ACUAAAAUAUUGUGUCUCUAAUAUCAGAAAUUCAGCUUUAAUAUAUUGGGUUUAACUCUUUGAA
AUUUAGAGUCUCCUUGAAAUACACAUGGGGGUGAUUUCCUAAACUUUAUUUCUUGUAAGGAUUU
AUCUCAGGGGUAACACACAAACCAGCAUCCUGAACCUCUAAGUAUGAGGACAGUAAGCCUUAAG

AAUAUAAAAUAAACUGUUCUUCUCUCUGCCGGUGGAAGUGUGCCCUGUCUAUUCCUGAAAUUGC
UUGUUUGAGACGCAUGAGACGUGCAGCACAUGAGACACGUGCAGCAGCCUGUGGAAUAUUGUCA
GUGAAGAAUGUCUUUGCCUGAUUAGAUAUAAAGACAAGUUAAACACAGCAUUAGACUAUAGAUC
AAGCCUGUGCCAGACACAAAUGACCUAAUGCCCAGCACGGGCCACGGAAUCUCCUAUCCUCUUGC
UUGAACAGAGCAGCACACUUCUCCCCCAACACUAUUAGAUGUUCUGGCAUAAUUUUGUAGAUAU
GUAGGAUUUGACAUGGACUAUUGUUCAAUGAUUCAGAGGAAAUCUCCUUUGUUCAGAUAAGUAC
ACUGACUACUAAAUGGAUUAAAAAACACAGUAAUAAAACCCAGUUUUCCCCUUAAAAAAAAAA
AAAAAAAAAAAAAAAAAGGGUUGGGUUUGGGUUGGG

Finally, it should be noted that the above embodiments are only used to facilitate those skilled in the art to understand the essence of the invention, and are not used to limit the protection scope of the invention.

## Claims

1. A method for enhancing the sustained release ability of a nucleic acid drug, comprising: adding DNA or RNA of a specific sequence at the 5' end and/or 3' end of the nucleic acid drug and/or inside the nucleic acid drug;
enhancing the sustained release ability of a nucleic acid drug means prolonging the sustained release time of the nucleic acid drug *in vivo* to more than 2 days and increasing the effective time of the nucleic acid drug *in vivo* to more than 2 days. Preferably, the sustained release time of the nucleic acid drug *in vivo* is prolonged to 2-20 days, and the effective time of the nucleic acid drug *in vivo* is increased to 2-20 days.

2. The method according to claim 1, wherein the nucleic acid drug may be an unmodified nucleic acid drug or a chemically modified nucleic acid drug.

3. The method according to claim 1 or 2, wherein the sequence length of the nucleic acid drug is ≥8 nt, preferably 8-5000 nt, more preferably 8-2000 nt, and most preferably 8-1000 nt.

4. The method according to claim 1 or 2, wherein the nucleic acid drug is a single-stranded DNA drug, a double-stranded DNA drug, a single-stranded RNA drug, a double-stranded RNA drug or a nucleic acid analog;
preferably, the nucleic acid drug is RNA nucleic acid aptamer, mRNA, ncRNA, antisense oligonucleotide ASO, DNA nucleic acid aptamer, or other DNA drug;
the ncRNA is miRNA, siRNA, shRNA, saRNA, sgRNA, piRNA, lncRNA, circRNA or other regulatory RNA.

5. The method according to claim 1 or 2, wherein:
the DNA or RNA of the specific sequence is the DNA or RNA with continuous G bases occurring at intervals; the continuous G bases are repeated 2-10 G bases; the number of intervals of the continuous G bases is 4-16, and the number of repeated G bases in each continuous G bases occurring at intervals is the same or different;
preferably, the DNA or RNA of the specific sequence also contains a spacer sequence occurring at intervals, which is a sequence of 1-6 arbitrary bases including G or not; the number of intervals of the spacer sequence is 3-15, and each of the spacer sequences occurring at intervals is the same or different.

6. The method according to claim 5, wherein the DNA or RNA of the specific sequence is a G-4 strand,
the formula of the G-quadruplex comprises Gₓ₁N_{y1}Gₓ₂N_{y2}Gₓ₃N_{y3}Gₓ₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3;
preferably, the G-quadruplex is:
G1 (SEQ ID NO.1): GGGTTGGGTTTGGGTTGGG, or
G1R (SEQ ID NO.9)_{:} GGGUUGGGUUUGGGUUGGG.

7. The method according to claim 6, wherein the DNA or RNA of the specific sequence is a sequence without any modification or a sequence with chemical modification;
preferably, the sequence of the chemical modification is:
(MOE-G)*(MOE-G)* (MOE-G)* (MOE-T)* TGGGTTTGGGTT(MOE-G)* (MOE-G)* (MOE-G)*
wherein, * represents a phosphorothioate linkage; MOE represents that the hydroxyl group (-OH) at the 2' position of ribose is replaced by methoxyethyl.

8. The method according to claim 5, wherein the association between DNA or RNA of the specific sequence and a nucleic acid drug is:
DNA of the specific sequence sustainedly releases a DNA drug, DNA of the specific sequence sustainedly releases a RNA drug, RNA of the specific sequence sustainedly release a DNA drug, or RNA of the specific sequence sustainedly releases a RNA drug;
preferably, the DNA of the specific sequence is linked with a DNA drug, so as to enhance its sustained release ability; or the RNA of the specific sequence is linked with a RNA drug, so as to enhance its sustained release ability.

9. Use of G-quadruplex in the preparation of a nucleic acid drug having enhanced sustained release ability,
the enhanced sustained release ability means prolonging the sustained release time of the nucleic acid drug *in vivo* to more than 2 days and increasing the effective time of the nucleic acid drug *in vivo* to more than 2 days; preferably, the sustained release time of the nucleic acid drug *in vivo* is prolonged to 2-20 days, and the effective time of the nucleic acid drug *in vivo* is increased to 2-20 days.

10. The application according to claim 9, wherein:
the formula of the G-quadruplex comprises Gₓ₁N_{y1}Gₓ₂N_{y2}Gₓ₃N_{y3}Gₓ₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3;
preferably, the G-quadruplex is:
G1 (SEQ ID NO.1)_{:} GGGTTGGGTTTGGGTTGGG, or
G1R (SEQID NO.9): GGGUUGGGUUUGGGUUGGG.

11. The application according to claim 10, wherein the nucleotide in the G-quadruplex is chemically modified,
preferably, the chemically modified G-quadruplex is:
(MOE-G)*(MOE-G)* (MOE-G)* (MOE-T)* TGGGTTTGGGTT(MOE-G)* (MOE-G)* (MOE-G)*
wherein, * represents a phosphorothioate linkage; MOE represents that the hydroxyl group (-OH) at the 2' position of ribose is replaced by methoxyethyl.

12. A nucleic acid drug containing G-quadruplex, which knocks down PCSK9 and reduces LDL, the nucleic acid drug is an ASO of one or more G-quadruplexes modified at both ends,
(1) the G-quadruplex is a nucleic acid fragment with formula comprising Gₓ₁N_{y1}Gₓ₂N_{y2}Gₓ₃N_{y3}Gₓ₄, wherein:
G represents guanine;
x1 - x4 each independently is an integer selected from 2-10, preferably 3-5, and most preferably 3;
each N independently represents any base (A, C, T, U), preferably T or U;
y1 - y3 each independently is an integer selected from 1-10, preferably 1-6, and more preferably 1-3;
preferably, the G-quadruplex is:
G1 (SEQ ID NO.1)_{:} GGGTTOGGTTTGGGTTGGG, or
G1R (SEQ ID NO.9)_{:} GGGUUGGGUUUGGGUUGGG;
further, the nucleotide of the G-quadruplex may be chemically modified. Preferably, the chemically modified G-quadruplex is:
(MOE-G)*(MOE-G)* (MOE-G)* (MOE-T)* TGGGTTTGGGTT(MOE-G)* (MOE-G)* (MOE-G)*
wherein, * represents a phosphorothioate linkage; MOE represents that the hydroxyl group (-OH) at the 2' position of the ribose is substituted by methoxyethyl;
(2) the ASO is any one of the following sequences:
PCSK9-ASO-1 (SEQ ID NO.83): AGCCACGTGGGCAGCAGCCTGTGA
PCSK9-ASO-2 (SEQ ID NO.84): TTCCACGTGGGCAGCAGCCTGTTT
PCSK9-ASO-3 (SEQ ID NO.85) : CGTAGACACCCTCACCCCCA
PCSK9-ASO-4 (SEQ ID NO.86): TTTAGACACCCTCACCCCTT
PCSK9-ASO-5 (SEQ ID NO.87) : TTTAGACACCCTCACCCCCAATT
PCSK9-ASO-6 (SEQ ID NO.88): CGTAGACACCCTCACCCCCAAAA
PCSK9-ASO-7 (SEQ ID NO.89)_{:} TTCATCCCGGCCGCTGACCTT
PCSK9-ASO-8 (SEQ ID NO.90): TTTCCCCAAAGTCCCCTT
PCSK9-ASO-9 (SEQ ID NO.91): TTCCACGTGGGCAGCAGCCTGTT
PCSK9-ASO-10 (SEQ ID NO.92): TTGCCACGTGGGCAGCAGCCTGTT
PCSK9-ASO-11 (SEQ ID NO.93): TTTCAGGGAACCAGGCTT
PCSK9-ASO-12 (SEQ ID NO.94): TTTCCTCAGGGAACCATT
PCSK9-ASO-13 (SEQ ID NO.95): TTGCTCCGGCAGCAGATTT
PCSK9-ASO-14 (SEQ ID NO.96): TTGGGATGCTCTGGGCTT
PCSK9-ASO-15 (SEQ ID NO.97): TTGCCTGTCTGTGGAATT
PCSK9-ASO-16 (SEQ ID NO.98): TTCTGGTCCTCAGGGAACCAGGCCTT

13. The nucleic acid drug according to claim 12, wherein the nucleic acid drug is a nucleic acid shown in any of the following sequences or any combination thereof:
(1) G1/PCSK9-ASO-1 (SEQ ID NO.59):
GGGTTGGGTTTGGGTTGGGAGCCACGTGGGCAGCAGCCTGTGAGGGTTGGGTTTGGGTTGGG
(2) G1/PCSK9-ASO-2 (SEQ ID NO.60):
GGGTTGGGTTTGGGTTGGGTTCCACGTGGGCAGCAGCCTGTTTGGGTTGGGTTTGGGTTGGG
(3) GI/PCSK9-ASO-3 (SEQ ID NO.61)_{:}
GGGTTGGGTTTGGGTTGGGCGTAGACACCCTCACCCCCAGGGTTGGGTTTGGGTTGGG
(4) G1/PCSK9-ASO-4 (SEQ ID NO.62):
GGGTTGGGTTTGGGTTGGGTTTAGACACCCTCACCCCTTGGGTTGGGTTTGGGTTGGG
(5) G1/PCSK9-ASO-5 (SEQ ID NO.63)_{:}
GGGTTGGGTTTGGGTTGGGTTTAGACACCCTCACCCCCAATTGGGTTGGGTTTGGGTTGGG
(6) G1/PCSK9-ASO-6 (SEQ ID NO.64):
GGGTTGGGTTTGGGTTGGGCGTAGACACCCTCACCCCCAAAAGGGTTGGGTTTGGGTTGGG
(7) G1/PCSK9-ASO-7 (SEQ ID NO.65):
GGGTTGGGTTTGGGTTGGGTTCATCCCGGCCGCTGACCTTGGGTTGGGTTTGGGTTGGG
(8) G1/PCSK9-ASO-8 (SEQ ID NO.66)_{:}
GGGTTGGGTTTGGGTTGGGTTTCCCCAAAGTCCCCTTGGGTTGGGTTTGGGTTGGG
(9) G1/PCSK9-ASO-9 (SEQ ID NO.67):
GGGTTGGGTTTGGGTTGGGTTCCACGTGGGCAGCAGCCTGTTGGGTTGGGTTTGGGTTGGG
(10) G1/PCSK9-ASO-10 (SEQ ID NO.68)_{:}
GGGTTGGGTTTGGGTTGGGTTGCCACGTGGGCAGCAGCCTGTTGGGTTGGGTTTGGGTTGGG
(11) G1/PCSK9-ASO-11 (SEQ ID NO.69):
GGGTTGGGTTTGGGTTGGGTTTCAGGGAACCAGGCTTGGGTTGGGTTTGGGTTGGG
(12) G1/PCSK9-ASO-12 (SEQ ID NO.70):
GGGTTGGGTTTGGGTTGGGTTTCCTCAGGGAACCATTGGGTTGGGTTTGGGTTGGG
(13) GI/PCSK9-ASO-13 (SEQ ID NO.71)_{:}
GGGTTGGGTTTGGGTTGGGTTGCTCCGGCAGCAGATTTGGGTTGGGTTTGGGTTGGG
(14) G1/PCSK9-ASO-14 (SEQ ID NO.72):
GGGTTGGGTTTGGGTTGGGTTGGGATGCTCTGGGCTTGGGTTGGGTTTGGGTTGGG
(15) G1/PCSK9-ASO-15 (SEQ ID NO.73):
GGGTTGGGTTTGGGTTGGGTTGCCTGTCTGTGGAATTGGGTTGGGTTTGGGTTGGG
(16) G1/PCSK9-ASO-16 (SEQ ID NO.74):
GGGTTGGGTTTGGGTTGGGTTCTGGTCCTCAGGGAACCAGGCCTTGGGTTGGGTTTGGGTTGGG
